(19) Europäisches Patentamt
European Patent Office
Office européen des brevets

(11) EP 1 666 458 A1

(12) **EUROPEAN PATENT APPLICATION**
published in accordance with Art. 158(3) EPC

(43) Date of publication:
**07.06.2006 Bulletin 2006/23**

(21) Application number: **04772118.8**

(22) Date of filing: **18.08.2004**

(51) Int Cl.:
***C07C 319/06*** *(1990.01)*
***C07C 323/58*** *(1990.01)*
***C07B 53/00*** *(1985.01)*
***C07D 233/76*** *(1985.01)*
***C12P 11/00*** *(1980.01)*

(86) International application number:
**PCT/JP2004/012157**

(87) International publication number:
**WO 2005/026110 (24.03.2005 Gazette 2005/12)**

(84) Designated Contracting States:
**AT BE BG CH CY CZ DE DK EE ES FI FR GB GR HU IE IT LI LU MC NL PL PT RO SE SI SK TR**

(30) Priority: **09.09.2003 JP 2003317402**

(71) Applicant: **KANEKA CORPORATION**
**Osaka-shi, Osaka 530-8288 (JP)**

(72) Inventors:
- **MATSUMOTO, Shingo, c/o Kaneka Corporation Takasago-shi, Hyogo 676-8688 (JP)**
- **MURAO, Hiroshi, c/o Kaneka Corporation Takasago-shi, Hyogo 676-8688 (JP)**
- **YAMAGUCHI, Takao, c/o Kaneka Corporation Takasago-shi, Hyogo 676-8688 (JP)**
- **IZUMIDA, Masashi, c/o Kaneka Corporation Takasago-shi, Hyogo 676-8688 (JP)**
- **UEDA, Yasuyoshi, c/o Kaneka Corporation Takasago-shi, Hyogo 676-8688 (JP)**

(74) Representative: **Vossius & Partner**
**Siebertstrasse 4**
**81675 München (DE)**

(54) **OPTICALLY ACTIVE 3,3 -DITHIOBIS(2-AMINO-2-METHYLPROPIONI C ACID) DERIVATIVE AND PROCESS FOR PRODUCING OPTICALLY ACTIVE 2-AMINO-3-MERCAPTO-2-METHYL-PROPIONIC ACID DERIVATIVE**

(57) The present invention provides a useful novel intermediate and a novel synthetic process that can highly prevent contamination by various impurities to an optically active R or S isomer of a 2-amino-3-mercapto-2-methylpropionic acid derivative or salt thereof useful as an intermediate for pharmaceuticals and the like and provides a process for easily and efficiently producing a high purity optically active R or S isomer of a 2-amino-3-mercapto-2-methylpropionic acid derivative or salt thereof on an industrial production scale. A process of producing a high purity 2-amino-3-mercapto-2-methylpropionic acid derivative or salt thereof includes reductively cleaving a sulfur-sulfur bond of an intermediate, which is a high purity optically active 3,3'-dithiobis(2-amino-2-methylpropionic acid) derivative substantially free of impurities. Thus, a resulting optically active 2-amino-3-mercapto-2-methylpropionic acid derivative can be produced without generating impurities as by-products which are difficult to remove.

Fig. 1

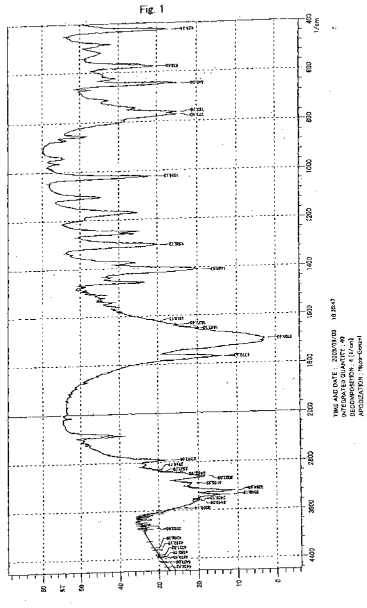

EP 1 666 458 A1

## Description

Technical Field

The present invention relates to optically active R or S isomers of 2-amino-3-mercapto-2-methylpropionic acid derivatives or salts thereof, which are useful as intermediates of medicines and the like, and to processes for producing optically active (2R,2'R) or (2S,2'S)-3,3'-dithiobis(2-amino-2-methylpropionic acid) derivatives or salts thereof, these compounds being useful as intermediates for pharmaceuticals and the like.

Background Art

The processes for producing optically active R or S isomers of 2-amino-3-mercapto-2-methylpropionic acid derivatives or salts thereof known to heretofore include the following:
1) A process of asymmetric methylation to an optically active thiazolidine compound obtained from optically active cysteine and pivalaldehyde (WO01/72702);
2) A process of asymmetric thiomethylation to an optically active oxazolone compound obtained from optically active alanine and benzaldehyde (J. Org. Chem., 1996, 61, 3350-3357);
3) A process of asymmetric methylation to an oxazolone compound obtained from cysteine and benzaldehyde (J. Org. Chem., 1992, 57, 5568-5573);
4) A process of conducting asymmetric bromomethylation of an optically active diketopiperazine compound obtained from optically active valine and alanine and replacing the bromine atom of the resulting compound by an alkali metal alkyl thiolate (Synthesis, 1983, 37-38);
5) A process of synthesizing optically active aziridine from optically active 2-methylglycidol obtained by Sharpless asymmetric oxidation of 2-methyl-2-propen-1-ol, and then reacting the resultant product with a thiol (J. Org. Chem., 1995, 60, 790-791); and
6) A process of methylating an aminomalonic acid derivative, conducting desymmetrization of the resulting product with porcine liver esterase (referred to as "PLE" hereinafter), and reacting the resulting unsymmetrical ester with a thioacetic acid alkali metal salt (J. Am. Chem. Soc., 1993, 115, 8449-8450).
However, 2-amino-3-mercapto-2-methylpropionic acid derivatives or the salts thereof have high water solubility and are difficult to extract with organic solvents. In particular, unsubstituted 2-amino-3-mercapto-2-methylpropionic acid or salts thereof have significantly high water solubility and cannot be extracted with organic solvents regardless of the value of the pH. Thus, they are extremely difficult to isolate. The only process for isolating the optically active R or S isomer of the 2-amino-3-mercapto-2-methylpropionic acid derivative or salt thereof described is the one described in the process (WO01/72702) under item 1) above, the process in which the aqueous hydrochloric acid solution of optically active 2-amino-3-mercapto-2-methylpropionic acid is concentrated to solidify as the hydrochloride thereof.
In each of the processes described in items 1) to 6) above, an appropriate auxiliary group is introduced to the starting materials used in stereoselective reaction in order to attain high stereoselectivity and to thereby obtain an optically active compound having a target configuration. Thus, in order to obtain the target optically active 2-amino-3-mercapto-2-methylpropionic acid derivative or salt thereof, the auxiliary group must be removed from the optically active intermediate obtained by the stereoselective reaction, i.e.,the optically active 2-amino-3-mercapto-2-methylpropionic acid derivative or salt thereof containing the auxiliary group. Here, the term "auxiliary group" refers to a substituent having an effect of improving the stereoselectivity in the course of the stereoselective reaction, or a substituent (known as "protecting group") that has an effect of preventing the undesirable action of a functional group that has an effect of inhibiting the reaction.
In the course of removing the auxiliary group from the optically active intermediate to produce an optically active 2-amino-3-mercapto-2-methylpropionic acid derivative or salt thereof, that an organic or an inorganic substance derived from the auxiliary group or a reagent used for removing the auxiliary group is generated as an impurity, i.e., a by-product. In some cases, an inorganic substance may be produced as the impurity, i.e., the by-product, in post-reaction treatment such as neutralization. Among these impurities, water-soluble impurities, such as inorganic substances, are extremely difficult to separate from the optically active 2-amino-3-mercapto-2-methylpropionic acid derivative having high water solubility. Thus, the optically active 2-amino-3-mercapto-2-methylpropionic acid derivative or salt thereof is subjected to the isolation process described in the process stated in the item 1) above while it still contains these water-soluble impurities. As a result, the water-soluble impurities exist together with the optically active 2-amino-3-mercapto-2-methylpropionic acid hydrochloride, and there is a tendency of the impurities contaminating the product optically active 2-amino-3-mercapto-2-methylpropionic acid hydrochloride. In the description below, an example of removing benzyl group, which is a thioether auxiliary group on the sulfur atom of optically active2-amino-3-benzylthio-2-methylpropionic acid obtained by the process described in item 3) above, so as to produce optically active 2-amino-3-mercapto-2-methylpropionic acid is described.
In general, the benzyl thioether auxiliary group is removed by a process (known as Birch reduction) that uses

metallic sodium and liquid ammonia. In this process, as the post-reaction treatment, unreacted metallic sodium is decomposed with alcohol or water and then the reduction products are extracted with an organic solvent to remove water-soluble sodium compounds, such as sodium hydroxide. However, when this process is applied to optically active 2-amino-3-benzylthio-2-methylpropionic acid, the resultant product, namely, 2-amino-3-mercapto-2-methylpropionic acid, cannot be extracted with an organic solvent regardless of the value of the pH and it has been necessary to add hydrochloric acid to the product still containing sodium compounds, such as sodium hydroxide, and then conduct the isolation process described in the process set forth in the item 1) above. As a result, it has been found that sodium chloride and the like generated between the sodium compound and the hydrochloric acid added solidify together with the optically active 2-amino-3-mercapto-2-methylpropionic acid hydrochloride, thereby contaminating the product optically active 2-amino-3-mercapto-2-methylpropionic acid hydrochloride.

The present inventors have also found that optically active 2-amino-3-mercapto-2-methylpropionic acid is relatively unstable, that impurities possibly derived from optically active 2-amino-3-mercapto-2-methylpropionic acid are readily produced as the by-products, that these impurities once generated are difficult to remove, and that it is difficult to prevent these impurities from contaminating the product. It is conceivable that impurities derived from optically active 2-amino-3-mercapto-2-methylpropionic acid are structural analogs that have parts similar to those of 2-amino-3-mercapto-2-methylpropionic acid. As is commonly known, structural analogs having a structure similar to the target compound behave like the target compound through the operational steps from the reaction to the post treatment and thus are easily mixed into the final products. In particular, in a case where the final product is pharmaceuticals, mixing of even a trace amount of an impurity may inflict a serious problem. Thus, it is vital that a process that can highly suppress generation of impurities and contamination be established.

As is discussed above, in the related art, no process that can suppress contamination by the above-described various impurities has been established, and there have been problems in the industrial process of producing the optically active 2-amino-3-mercapto-2-methylpropionic acid derivatives or salts thereof. Thus, establishment of an industrially practicable process for producing high-quality optically active 2-amino-3-mercapto-2-methylpropionic acid derivatives and salts thereof has been strongly desired.

## Disclosure of Invention

Under these circumstances, the present invention aims to develop a useful novel intermediate and a novel synthetic process that can highly prevent contamination by various impurities in the process of producing an optically active R or S isomer of a 2-amino-3-mercapto-2-methylpropionic acid derivative or salt thereof useful as an intermediate for pharmaceuticals and the like, and to provide a process for easily and efficiently producing a high purity optically active R or S isomer of a 2-amino-3-mercapto-2-methylpropionic acid derivative or salt thereof on an industrial production scale.

In aiming to achieve the object, the present inventors have vigorously investigated the process that can highly suppress contamination to the optically active 2-amino-3-mercapto-2-methylpropionic acid derivative or salt thereof by the above-described various impurities, namely, impurities derived from the auxiliary group, impurities derived from the reagent used to remove the auxiliary group, inorganic substances produced as by-products in the post-reaction treatment, such as neutralization, and impurities derived from the optically active 2-amino-3-mercapto-2-methylpropionic acid derivative. As a result, the inventors have found that an optically active 3,3'-dithiobis(2-amino-2-methylpropionic acid) derivative in which the auxiliary group (the protecting group) on the sulfur atom is a symmetrical disulfide protecting group can serve as an excellent intermediate that solves the above-described problems. In other words, the present inventors have found that a high purity optically active 3,3'-dithiobis(2-amino-2-methylpropionic acid) derivative can be easily obtained by removing impurities and that the resulting optically active 3,3'-dithiobis(2-amino-2-methylpropionic acid) derivative can be quantitatively converted to a corresponding optically active 2-amino-3-mercapto-2-methylpropionic acid derivative (target substance) by reductive cleavage of the sulfur-sulfur bond of the optically active 3,3'-dithiobis (2-amino-2-methylpropionic acid) derivative without generating by-products, i.e., impurities, which are difficult to remove.

The inventors have also found that, with this process, the contamination to the product optically active 2-amino-3-mercapto-2-methylpropionic acid derivative by the various impurities, which has been difficult to avoid according to the conventional process, can be minimized, and that the amounts of the impurities derived from the optically active 2-amino-3-mercapto-2-methylpropionic acid derivative can also be minimized. As is stated above, suppression of the contamination to the product optically active 2-amino-3-mercapto-2-methylpropionic acid derivative by various by-products, which have been difficult to remove by the conventional process, can be easily and efficiently achieved through the use of the optically active 3,3'-dithiobis(2-amino-2-methylpropionic acid) derivative.

The important intermediate of the present invention, i.e., the 3,3'-dithiobis(2-amino-2-methylpropionic acid) derivative described above, has three optical isomers, namely, a (2R,2'R) isomer, a (2S,2'S) isomer, and a meso isomer ((2R,2'S) or (2S,2'R)). The target optical isomers of the present invention are the (2R,2'R) isomer and the (25,2'S) isomer.

In particular, the present invention relates to a process for producing an optically active 2-amino-3-mercapto-2-methylpropionic acid derivative (1) represented by general formula (1) below or salt thereof:

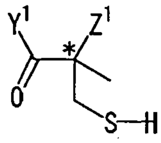

(1)

(wherein $Y^1$ is an unsubstituted hydroxyl group or a substituted or unsubstituted amino group and $Z^1$ is a substituted or unsubstituted amino group, or $Y^1$ and $Z^1$ together form a divalent group; and * is an asymmetric carbon), the process including:

reducing an optically active 3,3'-dithiobis(2-amino-2-methylpropionic acid) derivative represented by formula (2) below or salt thereof:

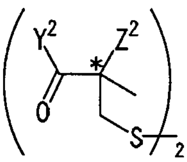

(2)

(wherein $Y^2$ and $Z^2$ respectively may be the same as or different from $Y^1$ and $Z^1$ above; $Y^2$ is an unsubstituted hydroxyl group or a substituted or unsubstituted amino group and $Z^2$ is a substituted or unsubstituted amino group, or $Y^2$ and $Z^2$ together form a divalent group; and * is an asymmetric carbon) so as to cleave the sulfur-sulfur bond; and converting $Y^2$ to $Y^1$ and/or $Z^2$ to $Z^1$ as necessary.

The present invention also relates to a process for producing the optically active 3,3'-dithiobis(2-amino-2-methylpropionic acid) represented by said general formula (2) or salt thereof, the process including oxidizing an optically active 3-mercapto-2-methylpropionic acid derivative represented by general formula (3) below or salt thereof

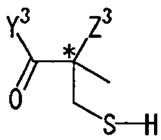

(3)

(wherein $Y^3$ and $Z^3$ may respectively be the same as or different from $Y^2$ and $Z^2$ above; $Y^3$ is an unsubstituted hydroxyl group or a substituted or unsubstituted amino group and $Z^3$ is a substituted or unsubstituted amino group, or $Y^3$ and $Z^3$ together form a divalent group; and * is an asymmetric carbon) to form a sulfur-sulfur bond between two molecules; and converting $Y^3$ to $Y^2$ and/or $Z^3$ to $Z^2$ as necessary.

The present invention also relates to a process for purifying an optically active 3,3'-dithiobis(2-amino-2-methylpropionic acid) derivative represented by said general formula (2) or salt thereof, the process including adjusting an aqueous medium solution containing the optically active 3,3'-dithiobis(2-amino-2-methylpropionic acid) derivative represented by said general formula (2) or salt thereof to be basic to separate and remove impurities from the solution.

The present invention also relates to a process for purifying the optically active 3,3'-dithiobis(2-amino-2-methylpropionic acid) derivative (2), the process including adjusting an aqueous medium solution containing the optically active 3,3'-dithiobis(2-amino-2-methylpropionic acid) derivative represented by said general formula (2) or salt thereof to be neutral to acidic so as to crystallize the optically active 3,3'-dithiobis(2-amino-2-methylpropionic acid) derivative (2), and removing impurities.

The present invention also relates to a process for purifying a salt with an acid of the optically active 3,3'-dithiobis(2-amino-2-methylpropionic acid) derivative represented by said general formula (2), the process including adjusting an aqueous medium solution containing the optically active 3,3'-dithiobis(2-amino-2-methylpropionic acid) derivative represented by said general formula (2) or salt thereof to be highly acidic so as to crystallize the salt with the acid of the optically active 3,3'-dithiobis(2-amino-2-methylpropionic acid) derivative (2), and removing impurities.

The present invention also relates to an optically active (2R,2'R) or (2S,2'S)-3,3'-dithiobis(2-amino-2-methylproplonic acid) derivative represented by formula (4) below or salt thereof:

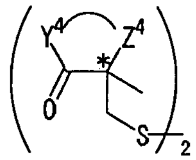

(4)

(wherein $-Y^4-Z^4-$ is a divalent group; and * is an asymmetric carbon) .

Best Mode for Carrying Out the Invention

The present invention will now be described in detail. First, the compounds of the present invention are described.

A 2-amino-3-mercapto-2-methylpropionic acid derivative represented by said general formula (1) is described first. Hereinafter, this derivative is also referred to as "compound (1)". In the compound (1), * indicates an asymmetric carbon, $Y^1$ and $Z^1$ may each independently be a monovalent group or may together form a divalent group. When $Y^1$ and $Z^1$ are each independently a monovalent group, $Y^1$ is an unsubstituted hydroxyl group or a substituted or unsubstituted amino group and $Z^1$ is a substituted or unsubstituted amino group.

When the $Y^1$ and $Z^1$ are each independently a monovalent group, in particular, examples of the substituent in the substituted amino group include aminocarbonyl group having 1 to 20 carbon atoms, alkoxycarbonyl group having 2 to 20 carbon atoms, acyl group having 1 to 20 carbon atoms, and a monovalent organic group having 1 to 20 carbon atoms. These substituents maybe substituted or unsubstituted. The substituted amino group may be monosubstituted or disubstituted. When the amino group is disubstituted, any combination of the above-described substituents can be used. Examples of the aminocarbonyl group having 1 to 20 carbon atoms include methylaminocarbonyl group, ethylaminocarbonyl group, and benzylaminocarbonyl group. Examples of the alkoxycarbonyl group having 1 to 20 carbon atoms are carbamate protecting groups for amino groups, such as methoxycarbonyl group, ethoxycarbonyl group, tert-butoxycarbonyl group, and benzyloxycarbonyl group. Examples of the acyl group having 1 to 20 carbon atoms are amide-type or imide-type protecting groups for amino groups and include formyl group, acetyl group, benzoyl group, and phthaloyl group. Examples of the monovalent organic group having 1 to 20 carbon atoms include alkyl groups having 1 to 20 carbon atoms, such as methyl group, ethyl group, n-propyl group, isopropyl group, n-butylgroup, isobutyl group, sec-butyl group, tert-butyl group, n-pentyl group, isopentyl group,and n-hexylgroup;aralkyl groups having 7 to 20 carbon atoms, such as benzyl group, 4-methylbenzyl group, 3-methylbenzyl group, 2-methylbenzyl group, 4-methoxybenzyl group, 3-methoxybenzyl group, 2-methoxybenzyl group, 1-phenylethyl group, 2-phenylethyl group, 1-(4-methylphenyl) ethyl group, 1-(4-methoxyphenyl) ethyl group, 3-phenylpropyl group, and 2-phenylpropyl group; and aryl groups having 6 to 20 carbon atoms, such as phenyl group, 1-naphthyl group, 2-naphthyl group, 4-methylphenyl group, 3-methylphenyl group, 2-methylphenyl group, 4-ethylphenyl group, 3-ethylphenyl group, 4-methoxyphenyl group, 3-methoxyphenyl group, 2-methoxyphenyl group,4-nitrophenylgroup,4-phenylphenylgroup, 4-chlorophenyl group, and 4-bromophenyl group.

The substituent contained in the substituted amino group may have a functional group as long as the essence of the present invention is not impaired (i.e., as long as the series of reactions is not particularly adversely affected). Examples of the functional group include amino group, hydroxyl group, phenyl group, aryl group, alkanoyl group, alkyl group, alkenyl group, alkynyl group, alkoxyl group, nitro group, and halogen atom.

When $Y^1$ and $Z^1$ are each independently a monovalent group, it is preferable that $Y^1$ and $Z^1$ are respectively an unsubstituted hydroxyl group and a substituted or unsubstituted amino group or are respectively an unsubstituted hydroxyl group and a substituted or unsubstituted ureido group ($-NHCONH_2$). More preferably, $Y^1$ and $Z^1$ are respectively an unsubstituted hydroxyl group and an unsubstituted amino group or respectively an unsubstituted hydroxyl group and an unsubstituted ureido group. Most preferably, $Y^1$ and $Z^1$ are respectively an unsubstituted hydroxyl group and an unsubstituted amino group.

When $Y^1$ and $Z^1$ together form a divalent group, $Y^1$ represents a substituted hydroxyl group or a substituted amino group, and $Z^1$ represents a substituted amino group. In other words, in this divalent group, the terminus at the $Y^1$ side is either an oxygen atom or a nitrogen atom, and the terminus at the $Z^1$ side is a nitrogen atom. No particular limitation is imposed other than the $Y^1$-side terminus (oxygen or nitrogen atom) and the $Z^1$-side terminus (nitrogen atom); however, the divalent group preferably forms a five-membered ring or six-membered ring by incorporating into the structure of the compound (1).

Specific examples of the divalent group (represented by $-Y^1-Z^1-$) include a substituted or unsubstituted ureylene group (-NHCONH-), a substituted or unsubstituted 1-oxa-3-aza-2-propanone-1,3-diyl group (-OCONH-), a substituted or unsubstituted 1-oxa-3-aza-propane-1,3-diyl group ($-OCH_2NH-$), a substituted or unsubstituted 1-oxa-3-aza-2-propene-1,3-diyl group (-OCH=N-), and a substituted or unsubstituted 1,4-diaza-2-butanone-1,4-diyl group (-

$NHCH_2CONH$-). Examples of the substituent of the divalent group include aminocarbonyl group having 1 to 20 carbon atoms, alkoxycarbonyl group having 1 to 20 carbon atoms, acyl group having 1 to 20 carbon atoms, and a monovalent organic group having 1 to 20 carbon atoms.

The substituent of the divalent group may have a functional group as long as the series of the reactions of the present invention is not particularly adversely affected. Examples of the functional group include amino group, hydroxyl group, phenyl group, aryl group, alkanoyl group, alkyl group, alkenyl group, alkynyl group, alkoxyl group, nitro group, and halogen atom.

When the $Y^1$ and $Z^1$ together form a divalent group, it is preferable that $Y^1$ and $Z^1$ together form a substituted or unsubstituted ureylene group (-NHCONH-), and in particular, an unsubstituted ureylene group.

Next, the 3,3'-dithiobis(2-amino-2-methylpropionic acid) derivative represented by said general formula (2) (hereinafter this derivative is also referred to as "compound (2) ") is described. In the compound (2), * is the same as above, and $Y^2$ and $Z^2$ may each independently be a monovalent group, may together form a divalent group, or may be the same as $Y^1$ and $Z^1$ describedabove. When $Y^2$ and $Z^2$ are each independently a monovalent group, $Y^2$ is an unsubstituted hydroxyl group or a substituted or unsubstituted amino group and $Z^2$ is a substituted or unsubstituted amino group.

The specific examples of $Y^2$ and $Z^2$ are the same as those described as the examples of $Y^1$ and $Z^1$.

When $Y^2$ and $Z^2$ are each independently a monovalent group, $Y^2$ and $Z^2$ are preferably respectively an unsubstituted hydroxyl group and a substituted or unsubstituted amino group, or an unsubstituted hydroxyl group and a substituted or unsubstituted ureido group ($-NHCONH_2$). More preferably, $Y^2$ and $Z^2$ are respectively an unsubstituted hydroxyl group and an unsubstituted amino group, or an unsubstituted hydroxyl group and an unsubstituted ureido group. Most preferably, $Y^2$ and $Z^2$ are respectively an unsubstituted hydroxyl group and an unsubstituted amino group.

When $Y^2$ and $Z^2$ together form a divalent group, it is preferable that $Y^2$ and $Z^2$ together form a substituted or unsubstituted ureylene group (-NHCONH-) and in particular, an unsubstituted ureylene group.

Thirdly, the 3-mercapto-2-methylpropionic acid derivative represented by said general formula (3) (hereinafter this derivative is also referred to as "compound (3)") is described. In the compound (3), * is the same as above; and $Y^3$ and $Z^3$ may each independently be a monovalent group, may together from a divalent group, or may be the same as $Y^2$ and $Z^2$ described above. When $Y^3$ and $Z^3$ are each independently a monovalent group, $Y^3$ is an unsubstituted hydroxyl group or a substituted or unsubstituted amino group, and $Z^3$ is a substituted or unsubstituted amino group.

Specific examples of $Y^3$ and $Z^3$ are the same as those described as the examples of $Y^1$ and $Z^1$.

When $Y^3$ and $Z^3$ are each independently a monovalent group, $Y^3$ and $Z^3$ are preferably an unsubstituted hydroxyl group and a substituted or unsubstituted amino group, respectively, or an unsubstituted hydroxyl group and a substituted or unsubstituted ureido group ($-NHCONH_2$), respectively. More preferably, $Y^3$ and $Z^3$ are an unsubstituted hydroxyl group and an unsubstituted amino group, respectively, or an unsubstituted hydroxyl group and an unsubstituted ureido group, respectively. Most preferably, $Y^3$ and $Z^3$ are an unsubstituted hydroxyl group and an unsubstituted amino group, respectively.

When $Y^3$ and $Z^3$ together form a divalent group, it is preferable that $Y^3$ and $Z^3$ together form a substituted or unsubstituted ureylene group (-NHCONH-), and in particular, an unsubstituted ureylene group.

According to the present invention, an effect of removing various by-products can be expected as described in detail below. Thus, the compound (3) containing various impurities, such as by-products, e.g., inorganic substances generated in the course of production of the compounds, and impurities derived from the compounds, may be used without any problem. Rather, the present invention is truly effective when the compound (3) containing impurities is used.

Fourthly, the optically active (2R,2'R) or (2S,2'S)-3,3'-dithiobis(2-amino-2-methylpropionic acid) derivative represented by said general formula (4) (hereinafter, this derivative is also referred to as "compound (4)") or salt thereof is described. The compound (4) is a useful compound as an intermediate for pharmaceuticals discovered in the present invention.

Both the (2R,2'R) optical isomer and the (2S,2'S) optical isomer of the compound (4) are included in the scope of the present invention.

In the compound (4), * is the same as above, and $-Y^4-Z^4-$preferably represents a divalent group that forms a five- or six-membered ring by incorporating into the structure of the compound (4). Specific examples of the divalent group include a substituted or unsubstituted ureylene group (-NHCONH-), a substituted or unsubstituted 1-oxa-3-aza-2-propanone-1,3-diyl group (-OCONH-), a substituted or unsubstituted 1-oxa-3-aza-propane-1,3-diyl group ($-OCH_2NH-$), a substituted or unsubstituted 1-oxa-3-aza-2-propene-1,3-diyl group (-OCH=N-), and a substituted or unsubstitutedl,4-diaza-2-butanone-1,4-diyl group ($-NHCH_2CONH-$). Examples of the substituent of the divalent group above include the aminocarbonyl group having 1 to 20 carbon atoms, the alkoxycarbonyl group having 2 to 20 carbon atoms, the acyl group having 1 to 20 carbon atoms, and the monovalent organic group having 1 to 20 carbon atoms mentioned above.

The substituent in the divalent group above may have a functional group as long as the series of reactions is not particularly adversely affected. Examples of the functional group include amino group, hydroxyl group, phenyl group, aryl group, alkanoyl group, alkyl group, alkenyl group, alkynyl group, alkoxyl group, nitro group, and halogen atom.

The group $-Y^4-Z^4-$ is more preferably a substituted or unsubstituted ureylene group (-NHCONH-), and most

preferably an unsubstituted ureylene group (-NHCONH-). An optically active (2R,2'R) or (2S,2'S)-3,3'-dithiobis(2-amino-2-methylpropionic acid) derivative, (a.k.a. (5R,5'R) or (55,5'S)-5,5-[dithiobis(methylene)]bis(5-methylhydantoin) derivative) represented by formula (5) below is preferred as the compound (4):

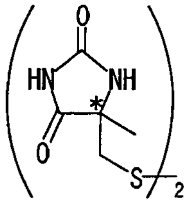
(5)

The individual reaction steps will now be described in detail.

A process of producing the 3,3'-dithiobis(2-amino-2-methylpropionic acid) derivative (2) or salt thereof used for the reduction in the present invention is described first.

The process of preparing the compound (2) is not particularly limited and any method can be used without any limitation. Among these processes, a process of forming a sulfur-sulfur bond between the molecules of the 3-mercapto-2-methylpropionic acid derivative (3) or salt thereof and converting $Y^3$ to $Y^2$ and/or $Z^3$ to $Z^2$ as necessary is preferable. Here, the meaning of the phrase "to convert $Y^3$ to $Y^2$ and/or $Z^3$ to $Z^2$" is, for example, to convert the substituted amino group to another substituted amino group by derivatization of the substituent, to convert the substituted amino group to an unsubstituted amino group by removal of the substituent, or to convert the unsubstituted amino group to a substituted amino group by introduction of a substituent. When $Y^3$ and $Z^3$ together form a divalent group, this phrase means that, for example, the divalent group is converted to another divalent group by derivatization or to a monovalent group indicated by $Y^2$ and $Z^2$ above. When conversion of $Y^3$ to $Y^2$ and/or $Z^3$ to $Z^2$ is performed, the conversion may take place after forming the sulfur-sulfur bond between the molecules of the compound (3) or salt thereof or before the formation of the sulfur-sulfur bond. The conversion of $Y^3$ to $Y^2$ and/or $Z^3$ to $Z^2$ may be conducted simultaneously with the formation of the sulfur-sulfur bond.

Examples of the process for forming the sulfur-sulfur bond between the molecules of the 3-mercapto-2-methylpropionic acid derivative (3) or salt thereof and converting $Y^3$ to $Y^2$ and/or $Z^3$ to $Z^2$ as necessary include a process for producing an optically active 3,3'-dithiobis(2-methylpropinionic acid) represented by formula (2a) below or salt thereof:

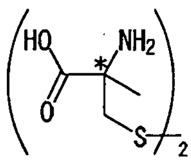
(2a)

(which is a compound represented by general formula (2) above with $Y^2$ representing an unsubstituted hydroxyl group and $Z^2$ representing an unsubstituted amino group, hereinafter, this compound is also referred to as the "compound (2a)"), the process including forming a sulfur-sulfur bond between molecules of 5-mercaptomethyl-5-methylhydantoin represented by formula (3b) below or salt thereof:

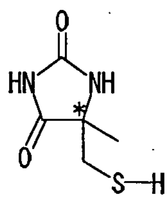
(3b)

(which is a compound represented by general formula (3) above with $Y^3$ and $Z^3$ together forming ureylene group (-NHCONH-), this compound is hereinafter also referred to as the "compound (3b)") to convert the compound (3b) to optically active 5,5'-[dithiobis(methylene)]bis(5-methylhydantoin) represented by formula (2'b) below or salt thereof:

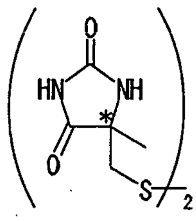

(2'b)

(this compound is hereinafter also referred to as the "compound (2'b)"), and then cleaving the hydantoin ring by hydrolysis of the ureylene group to convert the compound (2'b) to optically active 3,3'-dithiobis(2-methylpropionic acid) (2a) or salt thereof. Alternatively, the compound (2a) or salt thereof may be prepared by forming a sulfur-sulfur bond between molecules of 2-amino-3-mercapto-2-methylpropionic acid represented-by formula (3a) below or salt thereof:

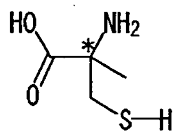

(3a)

(which is a compound represented by general formula (3) above with $Y^3$ representing an unsubstituted hydroxyl group and $Z^3$ representing an unsubstituted amino group, this compound is hereinafter also referred to as the "compound (3a)").

The compound (3a) and salt thereof described above may be obtained by hydrolysis of the ureylene group of the compound (3b) or salt thereof. Embodiments of producing the compound (2a) from the compound (3b) through the compound (3a) are also included in the scope of the present invention.

The process of forming a sulfur-sulfur bond between molecules of the compound (3) or salt thereof will now be described.

Examples of the process for forming the intermolecular sulfur-sulfur bond include a process involving reductive reaction of a halogenated sulfonyl compound, a halogenated sulfenyl compound, or a sulfinic acid compound; a process involving a reaction accompanied by cleavage of the cyano group of the thiocyanate compound; and a process involving oxidation of a thiol compound. The oxidation of a thiol compound easily and efficiently produces a high purity compound (2) or salt thereof and is thus particularly preferable. A process of producing a compound (2) or salt thereof by forming an intermolecular sulfur-sulfur bond by the oxidation of the compound (3) or salt thereof will now be described.

The oxidant used in the oxidation of the thiol compound is not particularly limited. Various oxidants may be used including oxygen (air) ; aqueous hydrogen peroxide; halogens such as bromine and iodine; hypohalous acids such as hypochlorous acid; sulfoxides such as dimethylsulfoxide; and transition metals such as manganese (IV) oxide, iron (III) chloride, and potassium hexacyanoferrate(III). The amount of the oxidant used cannot be categorically determined since it differs depending on the type of the oxidant. Preferably, a stoichiometric amount or more of the oxidant relative to the compound (3) is used from the standpoints of increasing the reaction rate and the yield.

Preferable examples of the oxidant are oxygen, aqueous hydrogen peroxide, halogens, and hypohalous acids. Among these, oxygen is particularly preferable since oxygen used as the oxidant can easily and efficiently produce a high-quality compound (2) or salt thereof by highly suppressing the side reaction. The oxidation usually has poor selectivity and the side reaction is sometimes difficult to control. In the oxidation of thiol compounds, for example, sulfonic acid and the like may be generated as the by-product, or excessive oxidation to the sulfoxides (thiosulfinate) may even occur. When oxygen is used as the oxidant, i.e., the oxygen oxidation or air oxidation, the oxidizing power is small and a practicable level of reaction rate may not be obtained. However, the investigation conducted by the present inventors shows that, contrary to the expectation, the compound (3) or salt thereof can be selectively converted to the target compound (2) or salt thereof, and that the conversion to the compound (2) or salt thereof can be carried out at a practical reaction rate even by the oxygen oxidation usually known to have small oxidation power. The details are described below by taking an example of oxidation by oxygen oxidation.

The process of introducing oxygen used as the oxidant is not particularly limited. Compressed air as an oxygen-containing gas may be introduced to a reactor using a steel cylinder or compressor. Naturally, oxygen may be directly fed or may be introduced as a mixed gas in which oxygen is diluted with an inert gas such as nitrogen, by using a steel cylinder of liquefied oxygen. In this case, it is possible to adjust the oxygen concentration in the reactor or in the mixed gas to be introduced so as to adequately control the amount of dissolved oxygen in the reaction solution.

In order to supply oxygen, the oxygen-containing gas (oxygen, compressed air, or mixed gas) may be introduced

in the gas phase or liquid phase of the reactor while evacuating from the vent (also known as aeration condition), or the internal pressure of the reactor may be adjusted to a predetermined pressure (not particularly limited but preferably normal pressure or high pressure) by supplying oxygen in an amount corresponding to the partial pressure consumed by the oxidation as necessary.

When oxygen is supplied by aeration, the reaction rate is greatly affected by the method of introducing the oxygen-containing gas (oxygen, compressed air, or mixed gas). From the standpoint of oxygen supply efficiency, a method of introducing the gas to the liquid phase is more efficient than the method of introducing the gas to the gas phase. The oxygen supply efficiency (oxidation rate) is also greatly affected by the manner of introduction. In order to efficiently supply oxygen, it is important that the contact efficiency between the oxygen-containing gas and the reaction solution be increased. In order to increase the contact efficiency with the reaction solution, it is preferable to break up bubbles generated by the introduction of the oxygen-containing air to the liquid phase as much as possible and to disperse the resulting broken bubbles so that the surface area of the bubbles is increased, thereby promoting migration of oxygen. Thus, in the case of an aeration nozzle having a single tube provided with a tip inserted into the reaction solution, the tip preferably includes a plurality of small holes. Moreover, it is particularly preferable to install a perforated-pipe distributor (sparger) at the bottom of the reactor below the stirring blade since the bubbles are broken up by the stirring blade during the course of rising from the bottom of the reactor.

With respect to the stirring during the reaction, excessively weak stirring tends to result in trapping of the gas (oxygen) at the gas-liquid interface at the surface of the reaction solution and in insufficient dispersion of the bubbles in the solution. Thus, in order to efficiently supply oxygen and to rapidly proceed the oxidation, a middle or higher level of stirring is preferable, in particular, the stirring is preferably as vigorous as possible. The stirring is not limited to stirring by blades, and it is effective to circulate the reaction solution. Examples of the method for circulation include a method (internal circulation inside the reactor) for generating upward flow and downward flow of the reaction solution by providing a partition or a baffle plate inside the reactor; and a method (external circulation) in which the reaction solution is circulated by a flow path disposed outside the reactor.

In the course of oxygen oxidation, there is a tendency that the reaction is promoted by basifying the reaction solution. In order to basify the reaction solution, the oxidation may be conducted in the presence of a basic substance or a basic substance may be added to the reaction solution. Although the basic substance is not particularly limited, use of inorganic bases is preferred. Examples of the inorganic bases include alkali metal hydroxides such as lithium hydroxide and sodium hydroxide; alkali metal carbonates such as sodium carbonate and potassium carbonate; and alkali metal hydrogen carbonates such as sodium hydrogen carbonate and potassium hydrogen carbonate.

The amount of the basic substance used is not particularly limited. For example, the molar amount of the basic substance is 0.1 to 10 times, preferably 0.5 to 5 times, and most preferably 0.5 to 2 times the molar amount of the compound (3). Note that when the basicity of the reaction solution to which the basic substance is added is indicated in terms of the pH, the pH is usually 8 or more, preferably 9 or more, and most preferably 10 or more. At an excessively high pH, side reaction such as decomposition of the compound (3) or compound (2) tends to occur. In order to inhibit the side reaction, the pH is usually 14 or less, preferably 13 or less, and most preferably 12 or less.

The reaction solvent is not particularly limited, and various solvents are usable. One type of solvent may be used alone as the reaction solvent, or a mixture of two or more types of solvent may be used. When an inorganic base is used as the basic substance, it is particularly preferable to use, as the reaction solvent, water alone or a mixture of water and another solvent.

The temperature of the reaction is not particularly limited but should be higher than the temperature (solidification point) at which the reaction solution does not solidify. The temperature is usually -10°C or higher, preferably 0°C or higher, and most preferably 10°C or higher. From the standpoint of increasing the reaction rate, it is preferable to increase the reaction temperature. The upper limit of the reaction temperature is not particularly limited but should not exceed the boiling point of the reaction solution. As a matter of course, it is possible to conduct reaction under the reflux conditions. Since the saturated concentration of oxygen in the reaction solution decreases with the increasing temperature, it is possible that the reaction rate is decreased at an excessively high temperature. The reaction temperature should be set to an appropriate level by taking into consideration these effects. The reaction temperature is usually 100°C or lower, preferably 80°C or lower, and most preferably 60°C or lower.

In the course of the oxygen oxidation, the reaction may be performed in the presence of an adequate amount of an oxidation catalyst or by adding an adequate amount of an oxidation catalyst so as to accelerate the reaction. In this manner, the reaction can be carried out at a lower temperature at a high rate; thus, the oxidation can be conducted at a moderate process temperature. The process temperature in such a case is usually 40°C or less, furthermore a practicable reaction rate can be maintained even at a temperature of 25°C or less.

The oxidation catalyst may be any catalyst that has an effect of promoting the reaction. Examples thereof are ionic compounds of heavy metals (transition metals) including divalent ionic iron compounds such as iron(II) chloride and iron(II) hydroxide and trivalent ionic iron compounds such as iron(III) chloride and iron(III) hydroxide; cupric compounds such as copper (II) sulfate and copper (II) hydroxide; and cobalt complexes such as phthalocyanine cobalt.

Among these, ionic iron compounds and cupric compounds are particularly preferred since they are easy to remove in the post-treatment described below. Ionic iron compounds are yet more preferable.

By the oxidation, an intermolecular sulfur-sulfur bond of the compound (3) or salt thereof can be formed at a high reaction conversion ratio. A conversion ratio of at least 95%, usually 98% or more, preferably 99% or more, and more preferably 99.9% or more can be expected.

As a result of the oxidation and the like, an intermolecular sulfur-sulfur bond can be formed between the molecules of the compound (3) or salt thereof. The $Y^3$ and $Y^2$ and/or $Z^3$ and $Z^2$ may be the same or different. When they are different, conversion of $Y^3$ to $Y^2$ and/or $Z^3$ to $Z^2$ is necessary to produce 3,3'-dithiobis(2-amino-2-methylpropionic acid) derivative (2) or salt thereof. Reaction for converting $Y^3$ to $Y^2$ and/or $Z^3$ to $Z^2$ may be conducted before or after the formation of the intermolecular sulfur-sulfur bond or simultaneously with the formation of the intermolecular sulfur-sulfur bond. The method for converting $Y^3$ to $Y^2$ and/or $Z^3$ to $Z^2$ will be described below.

The method for converting $Y^3$ to $Y^2$ and/or $Z^3$ to $Z^2$ is not particularly limited. For example, a method described in Protective Groups in Organic Synthesis, 2nd ed., John Willy & Sons (1991) may be used. The suitable method differs depending on the types of $Y^3$ and $Z^3$ Examples of the method include acid treatment, base treatment, hydrolysis, nitrous acid oxidation, and $Na/NH_3$ treatment. From the standpoint of operability, hydrolysis is preferred and hydrolysis using an acid is particularly preferred. An example of forming a sulfur-sulfur bond between two molecules of the compound (3b) or salt thereof to produce the compound (2'b) or salt thereof and then converting the resulting compound to the compound (2a) or salt thereof is described below.

First, in the manner described above, a sulfur-sulfur bond is formedbetweentwomolecules of the compound (3b) or salt thereof to produce the compound (2'b). In the case where the compound (2'b) or salt thereof is converted to the compound (2a) or salt thereof, the ureylene group (-NHCONH-, $-Y^3-Z^3-$) should be converted to the unsubstituted hydroxyl group ($Y^2$) and the unsubstituted amino group ($Z^2$). In particular, the compound (2'b) or salt thereof may be hydrolized under acidic conditions using hydrochloric acid or the like to cleave the hydantoin ring to thereby produce the compound (2a) or salt thereof.

In this manner, the compound (2) or salt thereof can be easily and efficiently produced by forming a sulfur-sulfur bond between molecules of the compound (3) or salt thereof and converting $Y^3$ to $Y^2$ and/or $Z^3$ to $Z^2$ as necessary.

Next, the method for purifying and isolating the compound (2) or salt thereof is described. The purification and isolation of the compound (2) is an important procedure of the present invention for preliminarily eliminating impurities or precursor thereof, which are difficult to remove once they become mixed with the target compound (1), at the stage of forming the compound (2). The impurities may be derived from any substance, and examples thereof include a reagent, such as an oxidation catalyst, used in the step of producing the compound (2) and derivatives therefrom, and substances contained in the starting materials, such as the compound (3), in trace amounts. The present inventors have searched for an efficient isolation and purification process that can satisfactorily remove the impurities and consequently found that the compound (2) and the impurities, in particular, the inorganic substances that are difficult to remove once they are mixed in the compound (1), can be efficiently separated by adequately adjusting the acidity or basicity of an aqueous medium solution containing the compound (2). Thus, the present inventors have established the process for purifying and isolating the compound (2). Examples of the inorganic substances include ionic compounds of representative metals, e.g., alkali metals, such as lithium, sodium, and potassium, and ionic compounds of heavy metals (transition metals) such as iron, copper, and cobalt that serve as the oxidation catalysts. In particular, the purification process of the present invention can efficiently separate the catalytic heavy metal ionic compounds which are no longer necessary after the oxidation, in particular, ionic iron compounds. The purification and isolation process can be roughly classified into the following three types:

1) A process for purifying a salt with a base of the compound (2), in which the aqueous medium solution is adjusted to be basic;
2) A process for purifying the compound (2), in which the aqueous medium solution is adjusted to be neutral to acidic; and
3) A process for purifying a salt with an acid of the compound (2), in which the aqueous medium solution is adjusted to be highly acidic.

The first purification process, in which the salt with the base of the compound (2) is purified by adjusting the aqueous medium solution containing the compound (2) or salt thereof to be basic so as to separate and remove the impurities from the solution, is described first.

According to the first purification process, an aqueous medium solution containing the compound (2) or salt thereof is adjusted to be basic so that the salt with the base of the compound (2) is dissolved in the aqueous medium and that impurities sparingly soluble in the aqueous medium are separated from the solution. In order to adjust the solution to be basic, the pH of the solution may be used as an index. The lower limit of the pH is usually 8 or more,

preferably 9 or more, and most preferably 10 or more from the standpoint of removing the impurities. The basic substance added to adjust the solution to be basic is not particularly limited. Any commercially available organic base and/or inorganic base may be used.

The impurity may be precipitated from the aqueous medium as a solid or separated as liquid depending on the type of the impurity. When the impurity is precipitated as a solid from the aqueous medium, the precipitated solid impurity can be separated and removed by a typical solid-liquid separation such as pressure filtration, vacuum filtration, or centrifugal filtration. For example, when the impurity is an ionic iron compound, such as iron (III) hydroxide, the impurity has a low solubility under basic conditions and can be precipitated as a solid. However, the ionic iron compound tends to form fine particles of extremely small particle size; hence, a filter element (the type, pore size, and material thereof) suited for collecting the fine particles of the impurity must be adequately set. For example, the pore size of the filter element is preferably about 1 μm or less and more preferably 0.5 μm or less. It is also preferable to use a filter aid to increase the filterability in collecting the fine particles of the impurity. The filter aid is not particularly limited, and diatomaceous earth, cellulose fibers, activated charcoal or the like may be used as the filter aid, for example. Among these, use of activated charcoal is preferable. The usage of the filter aid is not particularly limited, and both body feed and precoat filtration can be adequately used.

In contrast, when the impurity is separated as a liquid from the aqueous medium, a typical liquid-liquid separation, such as centrifugal separation, may be conducted as necessary to form two layers, i.e., an upper layer and a lower layer, that can be separated from each other, followed by removing the separated liquid as the impurity layer, for example. When the impurity is soluble in an organic solvent, the impurity may be removed by distribution in the organic solvent layer.

Furthermore, in a case where separation of the impurities is insufficient, it is possible to effectively adsorb and remove the impurities using a known adsorbent such as activated charcoal or activated clay. Use of activated charcoal as the filtering aid is preferable since not only the effect of increasing the filterability but also the effect of removing the impurity by adsorption can be expected.

Next, the second purification process for removing the impurities by adjusting the aqueous medium solution containing the compound (2) or salt thereof to be neutral to acidic to thereby crystallize a compound (2) in a free form is described.

In the second purification process, the aqueous medium solution containing the compound (2) or salt thereof is adjusted to be neutral to acidic so as to crystallize the compound (2) from the aqueous medium and dissolve the impurity in the aqueous medium. The crystallized compound (2) can be isolated and purified by a typical solid-liquid separation such as pressure filtration, vacuum filtration, or centrifugal filtration. For example, when the impurity is an ionic iron compound, such as iron(III) hydroxide, the solubility thereof under neutral to acidic conditions is higher than that under basic conditions; therefore, it is possible to dissolve the ionic iron compound in the aqueous medium. In order to adjust the solution to be neutral to acidic, an acidic substance may be added while using the pH of the solution as an index. The acidic substance added to adjust the solution to be neutral to acidic is not particularly limited and any typical acidic substance may be used. The upper limit of the pH is usually 9 or less, preferably 8 or less, and more preferably 7 or less from the standpoint of maintaining the recovery ratio of the compound (2). When the impurities are ionic iron compounds such as iron(III) hydroxide, the pH is usually 5 or less, preferably 4 or less, and more preferably 3 or less from the standpoint of removing the impurities. The lower limit of pH is usually 1 or more from the standpoint of maintaining the recovery ratio of the compound (2).

Lastly, the third purification process for removing the impurities by adjusting the solution containing the compound (2) or salt thereof and an aqueous medium to be highly acidic so as to crystallize the salt with the acid of the compound (2) is described.

In the third purification process, the aqueous medium solution containing the compound (2) or salt thereof is adjusted to be highly acidic to crystallize the salt with the acid of the compound (2) from the aqueous medium and to dissolve the impurities in the aqueous medium. The third purification method is suitably applied to a basic compound having a basic group or an amphoteric compound having both an acidic group and a basic group, e.g., optically active 3,3'-dithiobis(2-amino-2-methylpropionic acid) represented by said formula (2a). The crystallized salt with the acid of the compound (2) can be isolated and purified by a typical solid-liquid separation such as pressure filtration, vacuum filtration, or centrifugal filtration. For example, when the impurities are ionic iron compounds such as iron (III) hydroxide, the impurities have a higher solubility under highly acidic conditions than the salt with the acid of the compound (2). Thus, the ionic iron compounds can be dissolved in the aqueous medium. In order to adjust the solution to be acidic, an acidic substance may be added while using the equivalent weight relative to the compound (2) as an index. The acidic substance added to adjust the solution to be highly acidic is not particularly limited but is preferably a highly acidic substance having an ionization exponent (pK) of 1 or less. A highly acidic substance commonly used may be used. The equivalent weight of the acidic substance added cannot be uniformly defined since it differs depending on the type of acidic substance added. However, the equivalent weight is preferably 1 or more times, more preferably 5 or more times, and most preferably 10 or more times the compound (2) in terms of molar equivalents. When the pH of the solution is used as an index, the upper limit of the pH is preferably 1 or less and more preferably 0 or less from the standpoint of

maintaining the yield.

The three purification processes described above will now be specifically described by using the compound (2a) as an example.

A hydrochloride of the compound (3a) and iron (III) chloride are dissolved in water, and the pH of the solution is adjusted to 10 by addition of sodiumhydroxide. The reaction is then allowed to proceed while blowing air into the liquid phase to thereby quantitatively produce the compound (2a) . With the progress of the reaction, the pH of the reaction solution increases and reaches 11 at the completion of the reaction. Reddish-brown fine particles are precipitated as a result.

The resulting solution is filtered with a cellulose acetate membrane filter having a pore size of 0.45 $\mu$m or with cellulose fibers, activated charcoal, or the like as the filtering aid to suitably filter out the reddish-brown fine particles. The resulting filtrate containing the salt with the base of the compound (2a) thus has a satisfactorily low iron content. The iron component can be efficiently removed. This is an actual example of the first purification process.

Meanwhile, the compound (2a) is precipitated as a solid directly from the reaction solution by adding concentrated hydrochloric acid to the solution to control the pH of the solution to 3. Here, the iron components are adequately dissolved in the filtrate. Thus, by filtering and separating the compound (2a), the iron can be efficiently removed. This is the second purification process.

A hydrochloride of the compound (2a) is crystallized as a solid directly from the reaction solution by adding concentrated hydrochloric acid to the solution to adjust the pH to zero. Here, the iron components are adequately dissolved in the filtrate. Thus, by filtering and separating the hydrochloride of the compound (2a), the iron components can be efficiently removed. This is the third purification process.

The three purification processes described above may be used alone or in combination of two or three. The combination of the three purification processes is not particularly limited. Preferably, the first purification process is conducted before the second or third purification process. More preferably, the first purification process is conducted before the second purification process. By adjusting the pH as necessary, the resulting high-purity compound (2) or salt thereof can be freely converted to any form selected from the following three: a salt with the base of the compound (2), a free form of the compound (2), and a salt with the acid of the compound (2).

The high-purity compound (2) or salt thereof obtained by the above-described purification processes is used in the subsequent reductive reaction to adequately produce a high-purity compound (1), which is the object of the present invention.

Next, a process of cleaving the sulfur-sulfur bond of the 3,3'-dithiobis(2-amino-2-methylpropionic acid) derivative (2) or salt thereof by reductive reaction is described.

Any reductive reaction that can cleave the sulfur-sulfur bond can be employed without any limitation. For example, chemical reduction processes that use, as the reductants, alkali metals such as sodium; acids and metals such as zinc and tin; metal hydride reagents such as lithium aluminum hydride and boron sodium hydride; alkali metal sulfide such as potassium sulfide; and phosphine compounds, and electrolytic reduction processes may be employed. In all of these reduction processes, it is possible to cleave the sulfur-sulfur bond of the compound (2) or salt thereof to produce the 2-amino-3-mercapto-2-methylpropionic acid derivative (1).

As is previously stated in the description of the Related Art, the compound (1) is relatively unstable, and the impurities conceivably derived from the compound (1) are tend to be generated as the by-products. However, according to the reductive reaction of the present invention, it is possible to obtain a high purity compound (1) substantially free of the above-describedimpurities. Moreover, it has been found that the compound (1) is relatively unstable and tends to produce impurities as the by-products under the basic conditions. Thus, by adjusting the reductive reaction solution to be neutral to acidic, more preferably by maintaining the solution under neutral to acidic conditions through the reductive reaction process, it becomes possible to increase the stability of the compound (1) to more highly suppress the generation of the impurities as the by-products. The upper limit of pH is usually 9 or less, preferably 7 or less, and more preferably 5 or less.

The method for adjusting the reductive reaction solution to be neutral to acidic is not particularly limited. An acidic substance may be added before the initiation of the reductive reaction or during the reaction so that the reaction is conducted in the presence of the acidic substance, or after the completion of the reaction. When the neutral to acidic conditions are maintained through the reductive reaction process, it is preferable to allow the acidic substance to coexist before the initiation of the reductive reaction and to optionally add the acidic substance during or after the reaction as necessary. The acidic substance to be added is not particularly limited but is preferably a highly acidic substance. Specific examples thereof include inorganic acids such as hydrohalic acid, e.g., hydrochloric acid, sulfuric acid, sulfurous acid, andnitricacid; sulfonic acids such as methanesulfonic acid, p-toluenesulfonic acid, and o-, m-, or p-nitrobenzenesulfonic acid; and carboxylic acids such as trifluoroacetic acid. Among these, hydrohalic acid is preferred, and hydrochloric acid is particularly preferred.

Of the reduction methods described above, chemical reduction methods are convenient since they do not require special facilities as do the electrolytic reduction processes. In particular, the chemical reduction method that use

a metal such as zinc or tin with an acid, or a phosphine compound as the reductant can adequately use the compound (1) unstable under the basic conditions since reductive reaction can be relatively easily proceeded under neutral to acidic conditions according to these methods. In particular, a chemical reduction method that uses a phosphine compound as the reductant is preferable since removal of the by-products (impurities) derived from the reductant is easy. The operational conditions are described below by using a chemical reduction method that uses a phosphine compound as an example.

The phosphine compound usable as the reductant is not particularly limited but is usually preferably a tertiary phosphine compound. Preferable examples thereof include triarylphosphines such as substituted or unsubstituted triphenylphosphine and trialkylphosphines such as tri-n-butylphosphine and tri-n-octylphosphine. Triphenylphosphine is particularly preferable. Note that these phosphine compounds are converted to corresponding phosphine oxide compounds in the course of the reductive reaction.

The amount of the phosphine compound used cannot be uniformly defined since it differs depending on the type of the compound (2)or salt thereof or phosphine compound, and various conditions such as reaction temperature. From the standpoint of increasing the yield, the amount is preferably 1 molar equivalent or more relative to the compound (2). However, when the phosphine compound is used in large excess, the excessive phosphine compound used in the reductive reaction and the impurities, such as phosphine oxide compounds produced as the by-products in the reductive reaction, derived from the phosphine compound are increased. Thus, the load of removing these impurities in the post reaction treatment is also increased. From the standpoint of economy, the upper limit of the amount of the phosphine compound used is preferably 2 molar equivalents or less, more preferably 1.5 molar equivalents or less, and yet more preferably 1.3 molar equivalents or less relative to the compound (2).

The reaction solvent used for the reductive reaction using the phosphine compound is not particularly limited, and a typical solvent may be used. The reaction solvent may be water only, an organic solvent only, or a mixture of two or more of the solvents. When two or more solvents are mixed, the solvents may form a homogeneous phase or heterogeneous system. In particular, when a mixed solvent of water and an organic solvent is used as the reaction solvent, an organic solvent that is immiscible with the aqueous medium and that forms a two-phase system (heterogeneous system) is preferably selected since collection of the products and removal of the impurities are easy in the post-reaction treatment.

Examples of the organic solvent include aliphatic hydrocarbons such as hexane and heptane; aromatic hydrocarbons such as toluene and xylene; halogenated hydrocarbons such as methylene chloride and chloroform; ethers such as tert-butyl methyl ether, tetrahydrofuran, 1,4-dioxane, and dimethoxyethane; esters such as ethyl acetate and isopropyl acetate; ketones such as acetone, methyl ethyl ketone, and methyl isobutyl ketone; alcohols such as methanol, ethanol, isopropanol, and benzyl alcohol; and aprotic polar solvents such as acetonitrile, N,N-dimethylformamide, and dimethyl sulfoxide. Of these, aromatic hydrocarbons, halogenated hydrocarbons, and ethers immiscible with the aqueous medium are preferable. In particular, aromatic hydrocarbons are preferable. Among the aromatic hydrocarbons, toluene is particularly preferable.

The amount of the reaction solvent used cannot be uniformly defined since it differs depending on the type of the compound (2) or salt thereof or reaction solvent, and various conditions such as reaction temperature. Typically, the amount is 1,000 times the weight of the compound (2) or less, preferably 100 times or less, and most preferably 10 times or less from the standpoints of yield and the volume efficiency.

The temperature of the reductive reaction is not particularly limited but should be higher than the temperature (solidification point) at which the reaction solution does not solidify. From the standpoint of increasing the reaction rate, the higher reaction temperature is preferable. The upper limit of the reaction temperature is not particularly limited but should not exceed the boiling point of the reaction solution. As a matter of course, it is preferable to conduct the reaction under reflux conditions.

As is stated above, according to the reductive reaction of the present invention, the sulfur-sulfur bond of the compound (2) or salt thereof can be cleaved and the compound (2) or salt thereof can be substantially quantitatively converted to the compound (1) or salt thereof at a high reaction conversion ratio. The expected conversion ratio is at least 99% and preferably at least 99.9%.

The sulfur-sulfur bond of the compound (2) or salt thereof can be cleaved by the reductive reaction as described above. $Y^2$ and $Z^2$ may be the same as or different from $Y^1$ and $Z^1$, respectively. However, when $Y^2$ and $Z^2$ are different from $Y^1$ and $Z^1$, respectively, there is need to convert $Y^2$ to $Y^1$ and/or $Z^2$ to $Z^1$ to produce the compound (1) or salt thereof. Here, the meaning of the phrase "to convert $Y^2$ to $Y^1$ and/or $Z^2$ to $Z^1$" is, for example, to convert the substituted amino group to another substituted amino group by derivatization of the substituent, to convert the substituted amino group to an unsubstituted amino group by removal of the substituent, or to convert the unsubstituted amino group to a substituted amino group by introduction of a substituent. When $Y^2$ and $Z^2$ together form a divalent group, this phrase means that, for example, the divalent group is converted to another divalent group by derivatization or to a monovalent group indicated by $Y^1$ and $Z^1$ above. When conversion of $Y^2$ to $Y^1$ and/or $Z^2$ to $Z^1$ is performed, the conversion may take place after the completion of the reductive reaction of the compound (2) or salt thereof. Alternatively, $Y^2$ of the

compound (2) may be converted to $Y^1$, and/or $Z^2$ to $Z^1$ before the reductive reaction. Yet alternatively, $Y^2$ may be converted to $Y^1$ and/or $Z^2$ to $Z^1$ simultaneously with the reductive reaction.

Conversion of $Y^2$ to $Y^1$ and/or $Z^2$ to $Z^1$ can be conducted by the same method as that employed for converting $Y^3$ to $Y^2$ and/or $Z^3$ to $Z^2$.

The reductive reaction of the present invention can be equally applied to the compound (2), which is the impurity contained in the target compound (1) of the present invention. For example, in the case in which the reductant is deactivated by unexpected oxygen contamination during the reductive reaction and the reaction is thereby stopped before sufficiently achieving the expected conversion ratio, the reductive reaction of the present invention may be performed once again to complete the reaction so that the expected conversion ratio is attained. Another preferable example of the method for purifying the compound (1) containing the compound (2) as the impurity is a method for converting the compound (2), which is the impurity, to the compound (1) by the reductive reaction of the present invention. The content of the compound (2) as the impurity is not particularly limited. Even when the content of the compound (2) is 1% or more or 0.1% or more, the compound (2) content can be decreased by the reductive reaction of the present invention.

As is stated above, with the reductive reaction of the present invention, it is also possible to convert the compound (2) or salt thereof contained as the impurity in the compound (1) or salt thereof to the compound (1) or salt thereof at a high reaction conversion ratio by cleaving the sulfur-sulfur bond of the compound (2) or salt thereof.

Note that in the reductive reaction, as a precautionary measure, it is preferable to purge the interior of the reactor with inert gas such as nitrogen or argon to reduce the oxygen concentration in the reaction system from the standpoint of suppressing the inactivation of and the side reaction caused by the oxidation of the compound (1), which is the reduction product. The oxygen concentration in the reactor is usually 0.5% or less, preferably 0.2% or less, and more preferably 0.1% or less.

Next, the purification process for removing the impurities contained in the compound (1) obtained by the reductive reaction is explained. For example, in the case of producing the compound (1) by reducing the compound (2) by using a phosphine compound as the reductant, the phosphine compound, which is the reductant, and phosphine oxide compounds derived from the reductant are generated as impurities. In addition, impurities possibly derived from the compounds (1) and (2) are also produced as the by-products. Such impurities are frequently fat-soluble and have a high solubility in various organic solvents. Among these, the phosphine compound and the components (impurities such as phosphine oxide compounds) derived from the reductant are generally fat-soluble and exhibit high solubility in various organic solvents and a high distribution ratio during extraction. In contrast, the compound (1) has a high solubility in water regardless of the pH and is difficult to extract with various organic solvents.

Thus, the aqueous medium solution of the compound (1) containing the fat-soluble impurities maybe washed with an organic solvent immiscible with the aqueous medium so as to efficiently remove the fat-soluble impurities to the organic solvent layer. Thus, the aqueous medium solution containing the compound (1) or salt thereof can be purified.

The organic solvent used for the washing is not particularly limited and may be any common organic solvent. For example, reaction solvents usable in the reductive reaction are preferable as the organic solvent. Particularly preferable examples of the reaction solvent are organic solvents immiscible with the aqueous solvent and include aliphatic hydrocarbons such as hexane and heptane; aromatic hydrocarbons such as toluene and xylene; halogenated hydrocarbons such as methylene chloride and chloroform; ethers such as tert-butyl methyl ether and 1,4-dioxane; esters such as ethyl acetate and isopropyl acetate; and ketones such as methyl isobutyl ketone. Of these, aromatic hydrocarbons, halogenated hydrocarbons, and ethers are particularly preferable, and aromatic hydrocarbons are still more preferable. Among the aromatic hydrocarbons, toluene is particularly preferable.

The method of washing with the organic solvent is not particularly limited. In general, an organic solvent for washing is brought into contact with the aqueous medium solution containing the compound (1) to extract the fat-soluble impurities into the organic solvent phase, followed by separating and removing the resulting organic solvent layer. As a matter of course, the reaction solvent used in the reductive reaction may be directly used as the organic solvent for washing. Alternatively, the organic solvent may be directly added after the completion of the reductive reaction or added after the reaction solvent is distilled away (solvent substitution) as necessary.

The pH of the aqueous medium solution containing the compound (1) when brought into contact with the organic solvent is not particularly limited. It is preferable to adequately adjust the pH of the aqueous medium solution to increase the yield of the compound (1) and the impurity removal ratio. The adequate pH cannot be uniformly determined since it differs depending on the type of the compound (1). In the case of an amphoteric compound having both an acidic group and a basic group, it is preferable to adjust the pH to out of the range of 4 to 5, more preferably 3 or less.

An example of the amphoteric compound is an amino acid such as optically active 2-amino-3-mercapto-2-methylpropionic acid represented by formula (1a):

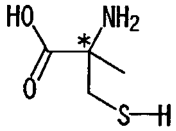

(1 a)

(which is a compound represented by said general formula (1) with $Y^1$ representing an unsubstituted hydroxyl group and $Z^1$ representing an unsubstituted amino group, hereinafter this compound is also referred to as "compound (1a)").

When the compound (1) is an acidic compound, the pH is preferably adjusted to 6 or higher, i.e., neutral to basic. An example of the acidic compound is a hydantoin derivative such as optically active 5-mercaptomethyl-5-methylhydantoin represented by formula (1b):

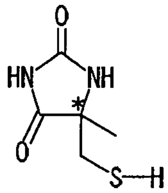

(1 b)

(which is the compound represented by said general formula (1) with $Y^1$ and $Z^1$ together forming ureylene group, hereinafter this compound is also referred to as the "compound (1b)").

As a result of the purification described above, an aqueous medium solution containing the compound (1) or salt thereof and from which various impurities are removed can be obtained. Accordingly, when the aqueous medium solution of the compound (1) produced by the present invention is used, solid of a high-quality salt with the acid of the compound (1) can be obtained by solidification and isolation according to the isolation method set forth in WO01/72702, which has not been possible by the conventional production process.

Alternatively, the compound (1) or salt thereof can be crystallized in the presence of an organic solvent. In particular, the compound (1) or salt thereof can be adequately crystallized by concentrating the water in the presence of an organic solvent to replace water with the organic solvent.

The compound (1) or salt thereof is not particularly limited. Examples thereof include the compound (1), a salt with an acid of the compound (1), and a salt with a base of the compound (1). A salt with an acid is particularly preferred.

The acid in the salt with the acid of the compound (1) is not particularly limited but is preferably highly acidic. In particular, inorganic acids such as hydrohalic acid, e.g., hydrochloric acid, sulfuricacid, sulfurousacid, andnitricacid; sulfonic acids such as methanesulfonic acid, p-toluenesulfonic acid, and o-, m-, or p-nitrobenzenesulfonic acid; and carboxylic acids such as trifluoroacetic acid are preferable. Of these, hydrohalic acid is preferred, and hydrochloric acid is particularly preferred.

Examples of the base in the salt with the base include amines such as ammonia, triethylamine, aniline, and pyridine.

In conducting the crystallization method, the aqueous medium solution containing the compound (1) or salt thereof may be preliminarily concentrated prior to the addition of the organic solvent. The type of the organic solvent for substitution is not particularly limited. An organic solvent azeotropic with water is preferred, and an organic solvent immiscible with water is more preferred.

The organic solvent is not particularly limited. Examples of the organic solvent include aliphatic hydrocarbons such as hexane and heptane; aromatic hydrocarbons such as toluene and xylene; halogenated hydrocarbons such as methylene chloride and chloroform; ethers such as tert-butyl methyl ether, 1,4-dioxane, and dimethoxyethane; and esters such as ethyl acetate and isopropyl acetate. Of these, aromatic hydrocarbons, esters, and ethers are particularly preferable. Aromatic hydrocarbons are yet more preferable since they have low dissolubility of the aqueous medium and the compound (1) or salt thereof and they are easy to recover and recycle. Among the aromatic hydrocarbons, toluene is particularly preferable.

In the substitution by the organic solvent, the distillation and feeding of the organic solvent may be conducted simultaneously (continuous method) or alternately over a plurality of times (batch method). The amount of the organic solvent for substitution is not uniformly defined since it differs depending on the type of the organic solvent, the degree of vacuum during the concentration, and the inner temperature. For example, where toluene is concerned, the amount is usually up to 100 times, preferably 50 times, and most preferably 10 times the total weight of the aqueous solution.

The concentration of the compound (1) or salt thereof in the course of crystallizing the compound (1) or salt

thereof by concentrating water in the presence of the organic solvent to conduct organic solvent substitution is preferably 0.1 wt% or more and more preferably 1 wt% or more. The upper limit is usually 70 wt% or less, preferably 50 wt% or less, and most preferably 30 wt% or less.

The final amount of water remaining after the removal of water to outside the system by the above-described operation is preferably 100 wt% or less relative to the compound (1) or salt thereof. From the standpoints of properties of the resulting crystals, filterability, crystal recovery ratio, and fluidity of the slurry, it is preferable to decrease water to 40 wt% or less.

The rate of evaporation for concentration depends on the shape and performance of the equipment, so it is not particularly defined. At a high evaporation rate, severe bubbling occurs, the resulting slurry exhibits extremely poor fluidity, and the crystals of the compound (1) tend to form aggregates/agglomerates. Thus, the evaporation rate per unit evaporation area and per unit time is preferably controlled to 1,000 $L/h \cdot m^2$ or lower, more preferably 600 $L/h \cdot m^2$ or lower, yet more preferably 300 $L/h \cdot m^2$ or lower, and most preferably 100 $L/h \cdot m^2$ or lower.

The degree of vacuum for concentration after the addition of the organic solvent differs depending on the type of the organic solvent but is usually 500 mmHg or less and preferably 200 mmHg orless. The lower limit is not particularly limited but is usually 0.1 mmHg or more.

The temperature for the concentration depends on the degree of vacuum and performance of the equipment but usually 0°C to 150°C, preferably 10°C to 100°C, and more preferably 30°C to 70°C so that the handling is easy and high-quality crystals can be obtained.

As is stated above, according to the present invention, the compound represented by said general formula (2) or salt thereof and the compound represented by said general formula (1) or salt thereof can be easily and efficiently produced from the compound represented by said general formula (3) or salt thereof on an industrial production scale while highly suppressing the contamination by impurities.

The combination of the compounds of the present invention is not particularly limited. The compounds (1), (2), and (3) can be freely combined to conduct the reaction. The most preferable combination of the compounds is, for example, to form a sulfur-sulfur bond between molecules of optically active 5-mercaptomethyl-5-methylhydantoin represented by said formula (3b) or salt thereof so as to produce the compound (2'b) or salt thereof, to hydrolyze the compound (2'b) or salt thereof to cleave the hydantoin ring to thereby produce the compound (2a) or salt thereof, and to then reduce the compound (2a) or salt thereof to cleave the sulfur-sulfur bond so as to produce the compound (1a) or salt thereof.

According to the present invention, it is possible to purify a low-quality compound represented by said general formula (3) or salt thereof or a low-quality compound represented by said general formula (1), containing the compound represented by said general formula (2) or salt as an impurity. The content of the compound (2), which is the impurity contained in the compound (3), is not particularly limited. Even when the content is 1% or more or 0. 1% or more, the content of the compound (2) can still be decreased by the above-described purification process.

For example, when the low-quality compound (3) or salt thereof contains water-soluble impurities, such as inorganic substances, that are difficult to remove from the compound (1), a high-quality compound (1) can be obtained by the process of the present invention, i.e., the process including converting the low-quality compound (3) to the compound (2) by oxidation, removing the water-soluble impurities by purification, and quantitatively reducing the resulting compound (2) to obtain the high-quality compound (1).

The content of the compound (2) as the impurity in the compound (3) is not particularly limited. Even at a compound (2) content of 1% or more or 0.1% or more, the compound (2) content can be reduced by the above-described purification process. In contrast, in the case of the low-quality compound (1) that does not contain impurities difficult to remove from the compound (1) other than the compound (2), the compound (2) contained as the impurity may be quantitatively reduced by the process of the present invention to produce the high-quality compound (1).

As is stated above, according to the present invention, a low-quality compound (1) or compound (3) can be easily and efficiently purified on an industrial production scale in all instances.

Next, the method for producing the optically active 3-mercapto-2-methylpropionic acid derivative (3) or salt thereof used in the production process of the present invention is described. The method for producing the compound (3) or salt thereof is not particularly limited, and various methods may be employed including those discussed as the examples of the related art. In view of the object of the present invention, a method suitable for industrial production is preferably used among these methods. An example of the preferable method is one described in WO03/106689, in which racemic 2-carbamoylamino-3-mercapto-2-methylpropionic acid or salt thereof is reacted with hydantoinase to selectively cyclize the D isomers and to thereby produce a D-5-mercaptomethyl-5-methylhydantoin derivative or salt thereof and L-2-carbamoylamino-3-mercapto-2-methylpropionic acid or salt thereof.

According to the above-described method, the optically active 3-mercapto-2-methylpropionic acid derivative (3) or salt thereof can be easily and efficiently produced on an industrial production scale. Thus, according to the present invention, the optically active 3,3'-dithiobis(2-amino-2-methylpropionic acid) derivative (2) or salt thereof and the optically active 2-amino-3-mercapto-2-methylpropionic acid derivative(1)orsalt thereof can be easily and efficiently produced from

the optically active 3-mercapto-2-methylpropionic acid derivative (3) or salt thereof on an industrial production scale while highly suppressing the contamination by the impurities.

EXAMPLES

The present invention will now be described in further detail by way of examples. It is to be understood that the present invention is not limited to these examples.

Various analyses in Examples were conducted under the following conditions: HPLC-1 [Column: Cosmosil 5C18-ARII (produced by Nacalai Tesque Inc.), mobile phase: potassium dihydrogen phosphate-phosphoric acid aqueous solution (pH 2.0)/acetonitrile = 97/3, flow rate: 1.0 ml/min, detection wavelength: 210 nm, column temperature: 40°C]; HPLC-2 [Column: Cosmosil 5C18-AR (produced by Nacalai Tesque Inc.), mobile phase: potassium dihydrogen phosphate-phosphoric acid·sodium heptanesulfonate aqueous solution (pH 2. 5) /acetonitrile = 95/5, flow rate: 1.0 ml/min, detection wavelength: 210 nm, column temperature: 40°C]; HPLC-3 [Column: Cosmosil 5C8-MS (produced by Nacalai Tesque Inc.) 150 mm × 4.6 mm I.D., mobile phase: potassium dihydrogen phosphate sodium hydroxide aqueous solution (pH 6.8)/acetonitrile = 1/1, flow rate: 1.0 ml/min, detection wavelength: 215 nm, column temperature: 40°C]; HPLC-4 [Column: CAPCELL PAK SCX (produced by Shiseido Co., Ltd.) 250 mm × 4.6 mm I.D., mobile phase: potassium dihydrogen phosphate phosphoric acid aqueous solution (pH 2.0) /acetonitrile = 95/5, flow rate: 0.3 ml/min, detection wavelength: 210 nm, column temperature: 40°C]; Chiral HPLC-1 [Column: CHIRALPAK AS (produced by Daicel), mobile phase:hexane/isopropanol/trichloroacetic acid = 9/1/0.01, flow rate: 0.5 ml/min, detection wavelength: 210 nm, and column temperature: 30°C]; Chiral HPLC-2 [Column: CHIRALPAKAS (produced by Daicel), mobile phase: hexane/ isopropanol = 9/1, flow rate: 1.0 ml/min, detection wavelength: 210 nm, column temperature: 30°C] ; Specific optical rotation [Light source: sodium lamp, cell length: 10 cm]; Iron content (atomic absorption spectrometry) [Atomization method: flame method (air-acetylene), detection wavelength: 248.3 nm (Fe)]; Sodium content (ion chromatography) [Column: TSKgel IC-Cation (produced by TOSOH), mobile phase: 2 mM nitric acid aqueous solution, flow rate: 1.0 ml/min, detector: electric conductivity, column temperature: 40°C]; IR [Equipment: single beam FT-IR, sample: KBr pellets].

The conversion ratio and the residual ratio in each Example are calculated by the following equations:

$$\text{conversion ratio} = \text{area of product}/(\text{area of starting material} + \text{area of product}) \times 100\ (\%)$$

$$\text{residual ratio} = \text{area of starting material}/(\text{area of starting material} + \text{area of product}) \times 100\ (\%)$$

(Reference Example 1) Preparation of racemic 5-tert-butylthiomethyl-5-methylhydantoin

Under a nitrogen atmosphere, 9.6 g of 5 wt% aqueous sodium hydroxide solution and 1.13 mL of tert-butyl mercaptan were combined at 0°C and stirred for 10 minutes. To the resulting mixture, 0.79 mL of chloroacetone was added, and the resulting mixture was warmed to room temperature and allowed to react for two hours. The reaction solution was buff yellow and separated into two phases.

To the reaction solution, 588 mg of NaCN, 2.77 gof $(NH_4)HCO_3$, and 3.1 mL of 30 wt% aqueous ammonia were added to prepare a homogeneous solution, followed by heating to 55°C to 60°C. After the solution was heated for 6 hours under stirring, the reaction solution was cooled to 0°C and combined with concentrated hydrochloric acid to adjust the pH to 7.0 to 7.6. White crystals thereby generated were filtered out, and 1.84 g of racemic 5-tert-butylthiomethyl-5-methylhydantoin was obtained as a result.

(Reference Example 2) Preparation of racemic 2-carbamoylamino-3-tert-butylthio-2-methylpropionic acid

In 75 g of 10% aqueous solution of sodium hydroxide, 4.77 g of racemic 5-tert-butylthiomethyl-5-methylhydantoin was dissolved and the solution was refluxed for 72 hours. The reaction solution was cooled to room temperature and then a portion of the reaction solution was sampled. A generation of racemic 2-amino-3-tert-butylthio-2-methylpropionic acid was confirmed from the sample by HPLC. The reaction solution was adjusted to a pH of 8 by concentrated hydrochloric

acid and heated to 70°C. To the resulting reaction solution, a solution of 2.07 g of potassium cyanate in 10 mL of distilled water was added dropwise over 20 minutes. After the completion of the addition, stirring was conducted for 5 hours, and a portion of the reaction solution was sampled and subjected to HPLC analysis (HPLC-1). Unreacted racemic 2-amino-3-tert-butylthio-2-methylpropionic acid was detected as a result. Thus, a solution of 4.14 g of potassium cyanate in 20 mL of distilled water was further added to the reaction solution dropwise over 20 minutes. Upon completion of the addition, stirring was conducted for 1 hour and the reaction solution was cooled to room temperature. The pH was adjusted to 2 with concentrated hydrochloric acid, and the solid deposits were filtered out. The resulting solid was washed with water and dried. As a result, 3.38 g of racemic 2-carbamoylamino-3-tert-butylthio-2-methylpropionic acid was obtained.

(Reference Example 3) Preparation of L-2-carbamoylamino-3-tert-butylthio-2-methylpropionic acid and D-5-tert-butylthiomethyl-5-methylhydantoin

In accordance with the culture method and preparation method of immobilized enzyme set forth in WO96/20275, the strain Bacillus sp. KNK245 (name of the depository institution to which the strain was deposited: National Institute of Bioscience and Human-Technology, Agency of Industrial Science and Technology, METI, Address: 1-1-3 Tsukuba-shi, Ibaraki Japan (zip code: 305), Date of accession: November 2, 1994, Accession number under which the strain was deposited with the depository institution: FERM BP-4863) was cultured and the cultured cells were collected. To an enzymatic solution obtained by ultrasonic disintegration of the cells, an anionic exchange resin serving as a support for immobilization, namely, Duolite A-568, was added to adsorb the enzyme, followed by cross-linking treatment with glutaric aldehyde. Immobilized hydantoinase was obtained as a result.

Next, to 15 mg of racemic 2-carbamoylamino-3-tert-butylthio-2-methylpropionic acid prepared in Reference Example 2, 1.5 ml of 0.1 M potassium phosphate buffer solution (pH = 7. 0) and 0.003 ml of 0.5 M aqueous manganese sulfate solution were added, the pH of the resulting solution was adjusted to 6.5 with 10N aqueous sodium hydroxide solution. To this solution, 200 mg (wet weight) of the immobilized hydantoinase obtained above was added, and the resulting mixture was allowed to react at 40°C for 48 hours under stirring. During the reaction, the pH was maintained at about 6.5 with 6N hydrochloric acid. The reaction solution was subjected to HPLC analysis (HPLC-1), and it was found that the residual ratio of 2-carbamoylamino-3-tert-butylthio-2-methylpropionic acid was 41%. The optical purity of 2-carbamoylamino-3-tert-butylthio-2-methylpropionic acid in the reaction solution was determined by HPLC analysis (chiral HPLC-1), and it was found that L-2-carbamoylamino-3-tert-butylthio-2-methylpropionic acid was produced in an optical purity of 96.7% ee. Meanwhile, the compound generated and precipitated during the enzymatic reaction described above was extracted with ethyl acetate and subjected to HPLC analysis (chiral HPLC-2). It was found that the compound was D-5-tert-butylthiomethyl-5-methylhydantoin.

L-2-Carbamoylamino-3-tert-butylthio-2-methylpropionic acid: $^1$H NMR (300 MHz, $CD_3OD$) δ: 3.22 (d,1H), 3.16 (d, 1H), 1.52 (s,3H), 1.29 (s,9H)

D-5-tert-Butylthiomethyl-5-methylhydantoin: $^1$H NMR (300 MHz, $CDCl_3$ with 3 drops of $CD_3OD$) δ: 2.90 (d,1H), 2.80 (d,1H), 1.49 (s,3H), 1.30 (s,9H).

(Reference Example 4) Preparation of

L-2-carbamoylamino-3-tert-butylthio-2-methylpropionic acid using bacteria of the genus Bacillus

Dry preserved cells of the strain Bacillus sp. KNK245 (FERM BP-4863) were inoculated in 100 ml of a liquid culture medium (10 g/l polypeptone, 10 g/l meat extract, 5 g/l yeast extract, pH: 7.5) sterilized at 120°C for 15 minutes in a 500 mL Sakaguchi flask, and shake culture was conducted at 45°C for 15 hours. Two milliliters of the cultured solution was inoculated in a culture medium in which 1 g/l of uracil and 20 mg/l of manganese chloride were added to the above-described culture components, and shake culture was performed at 45°C for 24 hours. Cells obtained from 15 ml of the cultured solution by centrifugal separation were suspended in 1.5 ml of 0.1 M potassium phosphate buffer solution (pH: 7.0), and combined with 150 mg of racemic 2-carbamoylamino-3-tert-butylthio-2-methylpropionic acid and 0.003 ml of 0.5 M aqueous manganese sulfate solution. Subsequently, the pH was adjusted to 6.5 with 10N aqueous sodium hydroxide solution. While maintaining the pH to about 6.5 with 6N hydrochloric acid, reaction was conducted at 40°C for 19 hours under stirring. The results of the HPLC analysis of the reaction solution showed that the residual ratio of 2-carbamoylamino-3-tert-butylthio-2-methylpropionic acid was 44%. The optical purity of 2-carbamoylamino-3-tert-butylthio-2-methylpropionic acid in the reaction solution was also determined by HPLC analysis (chiral HPLC-1). It was confirmed that L-2-carbamoylamino-3-tert-butylthio-2-methylpropionic acid

(Reference Example 5: Preparation of D-5-tert-butylthiomethyl-5-methylhydantoin)

In order to remove an impurity, i.e., 2-amino-3-tert-butylthio-2-methylpropionic acid, contained in 50 g of a mixture of the enzyme and D-5-tert-butylthiomethyl-5-methylhydantoin obtained by the method of Reference Example 3, 400 g of water was added. After the mixture was stirred, the insoluble components were filtered out and further washed with 200 g of water. 5 wt% aqueous sodium hydroxide solution (120 g) was added thereto, and the resulting mixture was stirred. The enzyme was filtered out as the insoluble component, and the pH of the filtrate was adjusted to 9 with concentrated hydrochloric acid. The crystals precipitated were filteredout, washed with water, and dried under a reduced pressure to obtain 19.7 g of a crude product as crystals. The crystals were analyzed by HPLC and were found to have a purity of 87.5 wt%. The optical purity thereof determined by HPLC analysis (chiral HPLC-2) was 97.6% ee.

(Reference Example 6) Preparation of D-5-mercaptomethyl-5-methylhydantoin

D-5-tert-Butylthiomethyl-5-methylhydantoin (4.38 g) obtained in Reference Example 4 was dissolved in 100 g of concentrated hydrochloric acid, and the solution was allowed to react at 80°C for 18.5 hours. After the solution was cooled to room temperature, the solution was concentrated to about half and combined with 30.5 g of 30 wt% aqueous sodium hydroxide solution to adjust the pH to 10. After extraction with ethyl acetate (100 mL $\times$ 3), the whole organic phase was concentrated to 10% of the total amount, and 30 mL of toluene was added the residue to deposit crystals. The crystals were filtered out, and 2.65 g of target D-5-mercaptomethyl-5-methylhydantoin was obtained as a result. The optical purity of this substance was measured by HPLC (chiral HPLC-2). No L isomer was detected as a result.

$^1$H NMR (400 MHz, MeOH-d4) $\delta$: 1.32 (s,3H), 2.60 (d,1.6 Hz, 1H), 2.72(d, 1.6 Hz, 1H)

(Reference Example 7: Preparation of D-2-amino-3-tert-butylthio-2-methylpropionic acid

To 80 g of a mixture of D-5-tert-butylthiomethyl-5-methylhydantoin and the enzyme obtained as in Reference Example 5, 150 mL of 10 wt% lithium hydroxide aqueous solution was added to dissolve the mixture. After filtering out the enzyme, the quantity of D-5-tert-butylthiomethyl-5-methylhydantoin in the mother liquid was determined by HPLC. As a result, 44.2 g of D-5-tert-butylthiomethyl-5-methylhydantoin was contained. To this solution, 54 g of lithium hydroxide and 51 g of distilled water were added, and the resulting mixture was refluxed under heating for 38 hours. After cooling to room temperature, solids generated were filtered. While maintaining the inner temperature of a mother liquid at about 20°C, 110 g of concentrated hydrochloric acid was added to adjust the pH to 6.7, followed by cooling to an inner temperature of 2°C and stirring for two hours. Subsequently, the solids generated were filtered, and vacuum-dried at 40°C for 24 hours. As a result, 34.9 g of dry crystals were obtained. The crystals were analyzed by HPLC (HPLC-1) and confirmed to be the title compound (purity: 96.7 wt%).

(Reference Example 8) Preparation of L-2-amino-3-tert-butylthio-2-methylpropionic acid

L-2-Carbamoylamino-3-tert-butylthio-2-methylpropionic acid (82.4 g) was dissolved in 630 g of 18% lithium hydroxide aqueous solution, and the resulting solution was refluxed for 41 hours under nitrogen. The solution was cooled to room temperature and filtered to remove the insoluble components. To the resulting solution, 180.1 g of concentrated hydrochloric acid was added to adjust the pH to 6. The solution was stirred for about 1 hour, then cooled to 4°C to 5°C, and again stirred for 1 hour. The generated crystals were filtered out, washed with water, and dried under a reduced pressure. As a result, 53.9 g of white solid was obtained.

$^1$H NMR (300 MHz, $D_2O$) $\delta$: 3.18 (d, 1H), 2.91 (d, 1H), 1.60 (s, 3H), 1.35(s, 9H)

(Reference Example 9) Preparation of L-2-amino-3-mercapto-2-methylpropionic acid hydrochloride

To 38.4 g of L-2-amino-3-tert-butylthio-2-methylpropionic acid obtained by the method of Reference Example 8, 345.3 g of concentrated hydrochloric acid was added, and the resultingmixture was refluxed for 24 hours to obtain an aqueous solution of L-2-amino-3-mercapto-2-methylpropionic acid hydrochloride.

The aqueous solution was concentrated to 67.5 g (degree of vacuum: 30 to 60 mmHg, temperature: 45°C) and combined with 206 g of toluene. The resulting mixture was subjected to vacuum concentration (degree of vacuum: 40 to 60 mmHg, temperature: 40°C, distillation rate: 107 L/h·m$^2$) so that the total weight was 109 g. Toluene (206 g) was added thereto to conduct concentration and the same operation was performed a total of six times to obtain 104 g of a toluene slurry of L-2-amino-3-mercapto-2-methylpropionic acid hydrochloride. The slurry contained 30 wt% of water relative to L-2-amino-3-mercapto-2-methylpropionic acid hydrochloride. Crystals were filtered, washed with toluene, and vacuum-dried to obtain 32.2 g of white solid of L-2-amino-3-mercapto-2-methylpropionic acid hydrochloride.

$^1$H NMR (300 MHz, $D_2O$) $\delta$: 3.18 (d, 1H), 2.89 (d, 1H), 1.60 (s, 3H) specific rotation: $[\alpha]^D{}_{20}$ = +8.77° (c1.15, $H_2O$)

The solid was confirmed to be the L stereoisomer since the sign of the specific rotation was coincident with that in the literature (Tetrahedron, 1993, 49, 2131-2138, WO98/38177).

(Example 1) Preparation of (5S,5'S)-5,5'-[dithiobis(methylene)]bis(5-methylhydantoin)

In 1,000 g of concentrated hydrochloric acid, 50 g of D-5-tert-butylthiomethyl-5-methylhydantoin obtained as in Reference Example 5 was dissolved, and the solution was allowed to react at 80°C for 18 hours to obtain an aqueous solution of D-5-mercaptomethyl-5-methylhydantoin. The residual ratio of D-5-tert-butylthiomethyl-5-methylhydantoin was 1% (HPLC-2).

The resulting reaction solution was concentrated to 208 g and combined with 133 g of 30 wt% aqueous sodium hydroxide solution at room temperature to adjust the pH to 9.0. To this solution, 13 mg (corresponding to 4 mg of iron) of iron trichloride was added, and the reaction was conducted under vigorous stirring with air bubbling at room temperature for 4 days to obtain 270 g of an aqueous solution containing 29.3 g of (5S,5'S)-5,5'-[dithiobis(methylene)]bis(5-methylhydantoin).

The iron content in the resulting aqueous solution was 16 ppm (corresponding to 4 mg of iron), and D-5-mercaptomethyl-5-methylhydantoin wasnot detected(lessthan 0.1% by HPLC-2).

(Example 2) Removing iron from (5S,5'S)-5,5'-[dithiobis(methylene)]bis(5-methylhydantoin)(activated charcoal treatment)

To 231 g (iron content: 4 mg) of the aqueous solution obtained in Example 1, 30 wt% aqueous sodium hydroxide solution was added to adjust the pH to 11.0. Reddish-brown crystals were precipitated as a result. The solution was filtered through 1.5 g of activate charcoal (produced by Takeda Pharmaceutical Company Limited: activated charcoal Shirasagi A) preliminarily washed with 0.01 M aqueous sodium hydroxide solution, and then further washed with 10 ml of 0.01 M aqueous sodium hydroxide solution to obtain 238 g of clear liquid. The iron content in the clear liquid was 0.02 ppm (corresponding to 0.006 mg of iron).

To 237 g of the clear liquid, 20 g of concentrated hydrochloric acid was added at room temperature to neutralize, thereby adjusting the pH to 1.8. Crystals precipitated were filtered, washed with water, and vacuum-dried to obtain 28.5 g of (5S,5'S)-5,5'-[dithiobis(methylene)]bis(5-methylhydantoin) as a white solid. The iron content in the crystals was 0.01 ppm (corresponding to 0.003 mg of iron).

$^1$H NMR (300 MHz, NaOD/$D_2$O) $\delta$: 3.15 (d, 1H), 3.05 (d, 1H), 1.35 (s, 3H)
$^{13}$C NMR (300 MHz, NaOD/$D_2$O) $\delta$: 196.9, 176.2, 68.3, 50.2, 25.6
IR (cm$^{-1}$, KBr) 1770.5, 1701.1, 1409.9, 1305.7, 1024.1, 773.4, 763.8, 648.0, 578.6, 426.2
$[\alpha]^D{}_{20}$ = +161.23° (c0.53, 1N NaOH)

(Example 3) Removing iron from (5S,5'S)-5,5'-[dithiobis(methylene)]bis(5-methylhydantoin) (filtration)

To 23.1 g of the aqueous solution (containing 0.4 mg of iron) obtained in Example 1, 30 wt% aqueous sodium hydroxide solution was added to adjust the pH to 11.0. Reddish-brown crystals were precipitated as a result. The solution was filtered through a membrane filter (pore size: 0.45 $\mu$m, made of cellulose acetate) to obtain 23.2 g of clear liquid. The iron content in the clear liquid was 0.09 ppm (corresponding to 0.002 mg of iron).

(Example 4) Crystallization of (5S,5'S)-5,5'-[dithiobis(methylene)] bis (5-methylhydantoin)

To 12 g of the aqueous solution (containing 0.2 mg of iron) obtained in Example 1, 10 g of concentrated hydrochloric acid was added at room temperature to neutralize, thereby adjusting the pH to 1.8. The precipitated crystals were filtered, washed with water, and vacuum-dried to obtain 14.6 g of (55,5'S)-5,5'-[dithiobis(methylene)]bis(5-methylhydantoin) as a white solid. The iron content in the resulting crystals was 98 ppm (corresponding to 0.1 mg).

(Example 5) Preparation of (2S,2'S)-3,3'-dithiobis(2-amino-2-methylpropionic acid)

In a 500 mL pressure reactor resistant to hydrochloric acid, 25 g of (5S,5'S)-5,5'-[dithiobis(methylene)]bis(5-methylhydantoin) obtained in Example 2 and 160 g of concentrated hydrochloric acid were enclosed and allowed to react for 36 hours in an oil bath at 120°C. HPLC analysis (HPLC-2) was conducted but (5S,5'S)-5,5'-[dithiobis(methylene)] bis(5-methylhydantoin) was not detected in the resulting reaction solution (less than 0.1%).

The resulting reaction solution (pH thereof was less than 0) was cooled to 3°C, and the precipitated crystals were filtered, washed with 25 mL of cold concentrated hydrochloric acid, and vacuum-dried to obtain 23.8 g of (2S,2'S)-3,3'-dithiobis(2-amino-2-methylpropionic acid) hydrochloride as a white solid.

$^1H$ NMR (300 MHz, NaOD/$D_2O$) δ: 3.31 (d, 1H), 2.92 (d, 1H), 1.30 (s,3H)
$^1H$ NMR (300 MHz, $D_2O$) δ: 3.59 (d,1H), 3.20 (d,1H), 1.63 (s,3H)
$^{13}C$ NMR (300 MHz, NaOD/$D_2O$) δ: 185.1, 61.7, 54.0, 28.8
$^{13}C$ NMR (300 MHz; $D_2O$) δ: 175.7, 63.2, 47.1, 24.9
$[\alpha]^D{}_{20}$ = +40.75° (c0. 53, 1N NaOH)
$[\alpha]^D{}_{20}$= -148.35° (c1.02, 1N HCl)

(Example 6) Preparation of D-2-amino-3-mercapto-2-methylpropionic acid by reduction of (2S,2'S)-3,3'-dithiobis(2-amino-2-methylproionic acid)

To 5.0 g of (2S,2'S)-3,3'-dithiobis(2-amino-2-methylpropionic acid) obtained in Example 5 and 6.4 g of triphenylphosphine, 50 g of toluene and 6.4 g of concentrated hydrochloric acid were sequentially added at room temperature under nitrogen stream. The mixture was then heated to 80°C. Subsequently, a total of 7.75 g of water was added in two divided portions to completely dissolve crystals of (2S,2'S)-3,3'-dithiobis(2-amino-2-methylpropionic acid). The reaction was further conducted for 24 hours to obtain 67 g of reaction solution containing 5.9 g of D-2-amino-3-mercapto-2-methylpropionic acid hydrochloride. The residual ratio of (25,2'S)-3,3'-dithiobis(2-amino-2-methylpropionic acid) in the resulting reaction solution was 0.3% (HPLC-3).

An aqueous solution (pH: 0) obtained by separating the toluene layer from this reaction solution was washed with 50 g of toluene four times. Triphenylphosphine and triphenylphosphine oxides were no longer detected as a result (lessthan 0.01% by HPLC-3) in the aqueous solution. Thus, aqueous D-2-amino-3-mercapto-2-methylpropionic acid hydrochloride solution with an optical purity of 99.6 area% was obtained.

The aqueous solution was concentrated (degree of vacuum: 30 to 60 mmHg, temperature: 45°C) to 13 g and combined with 40 g of toluene. Vacuum concentration was then conducted (degree of vacuum: 40 to 60 mmHg, temperature: 40°C, and distillation rate: 107 L/h·$m^2$) to bring the total weight to 15 g. Toluene (40 g) was further added thereto and concentration was conducted. The similar operation was carried out a total of five times. To the resulting solution, toluene was added to obtain 184 g of toluene slurry of D-2-amino-3-mercapto-2-methylpropionic acid hydrochloride. Crystals were filtered, washed with toluene, and vacuum-dried to obtain 5.9 g of D-2-amino-3-mercapto-2-methylpropionic acid hydrochloride as a white solid. As a result of HPLC analysis (HPLC-4), the purity of the resulting crystals of D-2-amino-3-mercapto-2-methylpropionic acid hydrochloride was 99.6 area%, and the content of (2S,2'S)-3,3'-dithiobis(2-amino-2-methylpropionic acid) was 0.3 area%.

$^1H$ NMR (300 MHz, $D_2O$) δ: 3.18 (d, 1H), 2.89 (d, 1H), 1.60 (s, 3H)
$[\alpha]^D{}_{20}$ = -8.76° (c1.01, $H_2O$)

(Example 7) Preparation of D-5-mercaptomethyl-5-methylhydantoin by reduction of (5S,5'S)-5,5'-[dithiobis(methylene)]bis(5-methylhydantoin)

To 5.9 g of (5S,5'S)-5,5'-[dithiobis(methylene)]bis(5-methylhydantoin) obtained in Example 4 and 6.4 g of triphenylphosphine, 50 g of toluene, 15.5 g of water, and 6.4 g of concentrated hydrochloric acid were sequentially added at room temperature under nitrogen stream, and the mixture was allowed to react for 24 hours at 80°C. The resulting reaction solution was analyzed by HPLC (HPLC-3). The residual ratio of (5S,5'S)-5,5'-[dithiobis(methylene)]bis(5-methylhydantoin) was 0.9%.

To this reaction solution, 30 wt% aqueous sodium hydroxide solution was added to adjust the pH to 9.0. An aqueous solution obtained by separating the toluene layer was washed with 50 g of toluene four times to obtain 22 g of an aqueous solution.

As a result of HPLC analysis, the aqueous solution contained 5.8 g of D-5-mercaptomethyl-5-methylhydantoin and the purity was 99.6 area%. Neither triphenylphosphine nor triphenylphosphine oxide was detected (less than 0.01% by HPLC-3).

To 21 g of the aqueous solution, concentrated hydrochloric acid was added at room temperature to adjust the pH to 2.8. Precipitated crystals were filtered, washed with water, and vacuum-dried to obtain 5.3 g of D-5-mercaptomethyl-5-methylhydantoin as a white solid. As a result of HPLC analysis (HPLC-2), the purity of the D-5-mercaptomethyl-5-methylhydantoin of the resulting crystals was 99.6 area%.

$^1H$ NMR (300 MHz, NaOD/$D_2O$) δ: 2.69 (s,2H), 1.33 (s,3H)
$[\alpha]^D{}_{20}$ = +98 .26° (c0. 50, 1N NaOH)

(Example 8) Preparation of (2R,2'R)-3,3'-dithiobis(2-amino-2-methylpropionic acid) (iodine oxidation)

In 72 g of water, 30 g of L-2-amino-3-mercapto-2-methylpropionic acid hydrochloride obtained by the same method as Reference Example 9 was dissolved, and 26.1 g of 30 wt% aqueous sodium hydroxide solution was added

thereto while maintaining the inner temperature at 25°C or less. While maintaining the inner temperature to 20°C to 25°C, 27.6 g of iodine was added in three divided portions over 30 minutes under vigorous stirring, and the reaction was further conducted for one hour. The resulting reaction solution was analyzed by HPLC (HPLC-2). As a result of the analysis, 0.6% of residual L-2-amino-3-mercapto-2-methylpropionic acid was detected.

The reaction solution was neutralized with concentrated hydrochloric acid at room temperature to adjust the pH to 2.8. The precipitated crystals were filtered, washed with water, and put under vacuum to obtain 23.0 g of (2R,2'R)-3,3'-dithiob.is(2-amino-2-methylpropionic acid) as a white solid.

$^1H$ NMR (300 MHz, NaOD/$D_2O$) $\delta$: 3.31 (d,1H), 2.92 (d,1H), 1.30 (s,3H) $[\alpha]^D{}_{20}$ = +217.53° (c1.00, 1N HCl)

(Example 9) Preparation of L-2-amino-3-mercapto-2-methylpropionic acid by reduction of (2R,2'R)-3,3'-dithiobis(2-amino-2-methylpropionic acid)

To 5.0 g of (2R,2'R)-3,3'-dithiobis(2-amino-2-methylpropionic acid) obtained in Example 8 and 6.4 g of triphenylphosphine, 50 g of tetrahydrofuran and 6.4 g of concentrated hydrochloric acid were sequentially added at room temperature under nitrogen stream. Reaction was conducted for 4 hours at 60°C to obtain 67 g of a reaction solution containing 6.3 g of L-2-amino-3-mercapto-2-methylpropionic acid hydrochloride. The residual ratio (by HPLC-3) of the (2R,2'R)-3,3'-dithiobis(2-amino-2-methylpropionic acid) in the resulting reaction solution was 0.3%.

(Example 10) Preparation of L-2-amino-3-mercapto-2-methylpropionic acid by reduction of (2R,2'R)-3,3'-dithiobis (2-amino-2-methylpropionic acid)

To 5.0 g of (2R,2'R)-3,3'-dithiobis(2-amino-2-methylpropionic acid) obtained in Example 8 and 6.4 g of triphenylphosphine, 50 g of toluene and 6.4 g of concentrated hydrochloric acid were sequentially added at room temperature under nitrogen stream. The resulting mixture was heated to 80°C. A total of 7.75 g of water was added in two divided portions to completely dissolve crystals of (2R,2'R)-3,3'-dithiobis(2-amino-2-methylpropionic acid). Subsequently, the reaction was conducted for 24 hours to obtain 67 g of reaction solution containing 5.9 g of L-2-amino-3-mercapto-2-methylpropionic acid hydrochloride. The residual ratio (by HPLC-3) of (2R,2'R)-3,3'-dithiobis(2-amino-2-methylpropionic acid) in the reaction solution was 0.3%.

An aqueous solution obtained by separating the toluene layer from the reaction solution was washed with 50 g of toluene four times. As a result, neither triphenylphosphine nor triphenylphosphine oxide was detected (less than 0.01% by HPLC-3). An aqueous solution of L-2-amino-3-mercapto-2-methylpropionic acid hydrochloride having a purity of 99.6 area% (HPLC-2) was thereby obtained.

The aqueous solution was concentrated to 13 g (degree of vacuum: 30 to 60 mmHg, temperature: 45°C), combined with 40 g of toluene, and subjected to vacuum concentration (degree of vacuum: 40 to 60 mmHg, temperature: 40°C, distillation rate: 107 $L/h \cdot m^2$) to bring the total weight to 15 g. Toluene (40 g) was added thereto and the resulting mixture was concentrated. The same operation was carried out a total of five times, and then toluene was added to obtain 184 g of a toluene slurry of L-2-amino-3-mercapto-2-methylpropionic acid hydrochloride. Crystals were filtered, washed with toluene, and vacuum-dried to obtain 6.2 g of L-2-amino-3-mercapto-2-methylpropionic acid hydrochloride as a white solid. As a result of the HPLC analysis (HPLC-4), the purity of the crystals of L-2-amino-3-mercapto-2-methylpropionic acid hydrochloride was 99.6 area%, and the content of (2R,2'R)-3,3'-dithiobis(2-amino-2-methylpropionic acid) was 0.3 area%.

$^1H$ NMR (300 MHz, $D_2O$ $\delta$: 3.18 (d,1H), 2.89 (d,1H), 1.60 (s,3H)

(Example 11) Preparation of (2R,2'R)-3,3'-dithiobis(2-amino-2-methylpropionic acid) (oxygen oxidation)

In 100 g of water, 10 g of L-2-amino-3-mercapto-2-methylpropionic acid hydrochloride obtained in Reference Example 9 was dissolved, and 12 g of 30 wt% aqueous sodium hydroxide solution was added while maintaining the inner temperature to 25°C or less. The solution was then allowed to react under vigorous stirring under air bubbling while maintaining the inner temperature at 20°C to 25°C. Three days were required to complete the reaction. The reaction solution was neutralized with 10 g of concentrated hydrochloric acid at room temperature to adjust the pH to 2.8. Crystals precipitated were filtered, washed with water, and vacuum-dried to obtain 6. 6 g of (2R,2'R)-3,3'-dithiobis(2-amino-2-methylpropionic acid) as a white solid. As a result of HPLC analysis (HPLC-2), the L-2-amino-3-mercapto-2-methylpropionic acid content was 0.3%.

$^1H$ NMR (300 MHz, NaOD/$D_2O$) $\delta$: 3.31 (d,1H), 2.92 (d,1H), 1.30 (s,3H)

(Example 12) Preparation of (2R,2'R)-3,3'-dithiobis(2-amino-2-methylpropionic acid) (iron-catalyzed oxygen oxidation)

In 30 g of water, 10 g of L-2-amino-3-mercapto-2-methylpropionic acid hydrochloride obtained in Reference

Example 9 and 1 mg (corresponding to 0.3 mg of iron) of iron trichloride were dissolved. To the solution, 9 g of 30 wt% aqueous sodium hydroxide solution was added while maintaining the inner temperature at 25°C or less. Reaction was conducted under vigorous stirring with air bubbling while maintaining the inner temperature to 20°C to 25°C. It required 30 hours to complete the reaction. With the progress of the reaction, products were precipitated, and the reaction solution thereby formed slurry having a pH of 9.4.

To this reaction solution, 30 wt% aqueous sodium hydroxide solution was added to adjust the pH to 11.0. The precipitated products were dissolved as a result. The resulting aqueous solution (51 g) contained 7.6 g of (2R,2'R)-3,3'-dithiobis(2-amino-2-methylpropionic acid). Furthermore, trace amounts of reddish-brown crystals remained undissolved.

The iron content in the aqueous solution was 12 ppm (corresponding to 0.6 mg).
L-2-Amino-3-mercapto-2-methylpropionic acid was not detected (less than 0.1% by HPLC-2).

(Example 13) Removing iron from (2R,2'R)-3,3'-dithiobis(2-amino-2-methylpropionic acid) (treatment with activated charcoal as filtering aid)

To 10 g (containing 0.1 mg of iron) of the aqueous solution obtained in Example 12, 100 mg of activated charcoal (produced by Takeda Pharmaceutical Company Limited: activated charcoal Shirasagi A) preliminarily washed with 0.01 M aqueous sodium hydroxide solution was added, and the mixture was filtered through a filter paper (pore size: 0.8 μm, composed of cellulose) and washed with 1 ml of 0.01 M aqueous sodium hydroxide solution to obtain 11 g of a clear liquid. The iron content in the clear liquid was 0.1 ppm (corresponding to 0.001 mg of iron).

Atroomtemperature, 10 g of the clear liquidwas neutralized with concentrated hydrochloric acid to adjust the pH to 1.8. Crystals precipitated were filtered, washed with water, and vacuum-dried to obtain 1.3 g of (2R,2'R)-3,3'-dithiobis(2-amino-2-methylpropionic acid) as a white solid. The iron content in the crystals was 0.8 ppm (corresponding to 0.001 mg).

$^{1}H$ NMR (300MHz, $NaOD/D_2O$) δ: 3.15 (d, 1H), 3.05 (d, 1H), 1.35 (s,3H)
$^{13}C$ NMR (300 MHz, $NaOD/D_2O$) δ: 196.9, 176.2, 68.3, 50.2, 25.6
$[\alpha]^{D}_{20}$ = +216. 43° (c1. 04, 1N HCl)

(Example 14) Removing iron from (2R,2'R)-3,3'-dithiobis(2-amino-2-methylpropionic acid) (treatment with cellulose as filtering aid)

To 10 g (containing 0.1 mg of iron) of the aqueous solution obtained in Example 12, 100 mg of powdered cellulose (produced by Nippon Paper Chemicals, KC Flock) preliminarily washed with 0.01 M aqueous sodium hydroxide solution was added, and the resulting mixture was filtered through a filter paper (pore size: 0.8 μm, composed of cellulose) and washed with 1 ml of 0.01 M aqueous sodium hydroxide solution to obtain 11 g of clear liquid. The iron content in the clear liquid was 0.1 ppm (corresponding to 0.001 mg of iron).

(Example 15/Comparative Example 1) Removing iron from (2R,2'R)-3,3'-dithiobis(2-amino-2-methylpropionic acid) (filter treatment)

10 g of the aqueous solution (containing 0.1 mg of iron) obtained in Example 12 was filtered through a membrane filter (pore size: 0.45 μm, composed of cellulose acetate) and washed with 1 ml of 0.01 M aqueous sodium hydroxide solution to obtain 11 g of clear liquid. The iron content in the clear liquid was 0.1 ppm (corresponding to 0.001 mg of iron) (Example 15).
Meanwhile, 10 g of the aqueous solution (containing 0.1 mg of iron) obtained in Example 11 was filtered through a filter paper (pore size: 4 μm, composed of cellulose), and reddish-brown crystals passed through the filter. The filter paper was further washed with 1 ml of 0.01 M aqueous sodium hydroxide solution to obtain 11 g of turbid liquid. The iron content in the turbid was 12 ppm (corresponding to 0.1 mg of iron) (Comparative Example 1).

(Example 16) Crystallization of (2R,2'R)-3,3'-dithiobis(2-amino-2-methylpropionic acid)

10 g of the aqueous solution (containing 0.1 mg of iron) obtained in Example 12 was neutralized with concentrated hydrochloric acid to adjust the pH to 1.8, and the precipitated crystals were filtered, washed with water, and vacuum-dried to obtain 1.3 g of (2R,2'R)-3,3'-dithiobis(2-amino-2-methylpropionic acid) as a white solid. The iron content in the resulting wet crystals was 2 ppm (corresponding to 0.05 mg of iron).

(Reference Example 10) Preparation of L-2-amino-3-mercapto-2-methylpropionic acid hydrochloride

The standard of the iron content in the industrial-grade concentrated hydrochloric acid is 20 ppm or less. Usually

industrial-grade concentrated hydrochloric acid with an iron content of 0.2 ppm or less is available. The reference example described below concerns an example of producing L-2-amino-3-mercapto-2-methylpropionic acid hydrochloride by using concentrated hydrochloric acid having high iron content.

To 7.8 g of L-2-amino-3-tert-butylthio-2-methylpropionic acid obtained by the process of Reference Example 8, 64.4 g of concentrated hydrochloric acid (iron content: 4 ppm, corresponding to 0.3 mg) was added, and the resulting mixture was refluxed for 24 hours to obtain 70 g of an aqueous solution of L-2-amino-3-mercapto-2-methylpropionic acid hydrochloride. The iron content in the resulting reaction solution was 4 ppm (corresponding to 0.3 mg).

This aqueous solution (69 g) was concentrated to 13 g (degree of vacuum: 30 to 60 mmHg, temperature: 45°C), combined with 41 g of toluene, and subjected to vacuum concentration (degree of vacuum: 40 to 60 mmHg, temperature: 40°C) to bring the total weight to 22 g. To this, 41 g of toluene was added and the resulting solution was concentrated. The same operation was carried out a total of six times to obtain 21 g of a toluene slurry of L-2-amino-3-mercapto-2-methylpropionic acid hydrochloride. Crystals were filtered, washed with toluene, and vacuum-dried to obtain 6.4 g of L-2-amino-3-mercapto-2-methylpropionic acid hydrochloride as a white solid.

The iron content in the resulting crystals was 52 ppm (corresponding to 0.3 mg), and the content of (2R,2'R)-3,3'-dithiobis(2-amino-2-methylpropionic acid) was 0.7% (HPLC-2). It should be noted here that L-2-amino-3-mercapto-2-methylpropionic acid hydrochloride tends to show degraded stability with higher iron content.

<u>(Example 17) Preparation of (2R,2'R)-3,3'-dithiobis(2-amino-2-methylpropionic acid)</u>

In 15 g of water, 5 g (containing 0.3 mg of iron) of L-2-amino-3-mercapto-2-methylpropionic acid hydrochloride obtained in Reference Example 10 was dissolved, and 4.5 g of 30 wt% aqueous sodium hydroxide solution was added thereto while maintaining the inner temperature at 25°C or less. The reaction was carried out for 60 hours under vigorous stirring with air supplied to the gas phase while maintaining the inner temperature at 20°C to 25°C. The resulting reaction solution was subjected to HPLC analysis (HPLC-2), and as a result, 6.3% of residual L-2-amino-3-mercapto-2-methylpropionic acid was detected.

Subsequently, while air is blown into the liquid, reaction was conducted for 24 hours under vigorous stirring. The resulting reaction solution had a pH of 13.3 and a very trace amount of reddish-brown crystals were precipitated. As a result of HPLC analysis (HPLC-2), L-2-amino-3-mercapto-2-methylpropionic acid was not detected (less than 0.1%).

The reaction solution was neutralized with concentrated hydrochloric acid to adjust the pH to 11.2 and aged overnight under vigorous stirring. Reddish-brown crystals were precipitated as a result.

The resulting aqueous solution (22 g) contained 3.7 g of (2R,2'R)-3,3'-dithiobis(2-amino-2-methylpropionic acid). Moreover, the iron content in the resulting aqueous solution was 14 ppm (corresponding to 0.3 mg).

<u>(Example 18) Removing iron from (2R,2'R)-3,3'-dithiobis(2-amino-2-methylpropionic acid) (treatment with activated charcoal as filterin aid)</u>

10 g of the aqueous solution (containing 0.14 mg of iron) obtained in Example 17 was filtered through 100 mg of activated charcoal (produced by Takeda Pharmaceutical Company Limited: activated charcoal Shirasagi A) preliminarily washed with 0.01 M aqueous sodium hydroxide solution, and further washed with 1 ml of 0.01 M aqueous sodium hydroxide solution to obtain 11 g of clear liquid. The iron content in the clear liquid was 0.9 ppm (corresponding to 0.01 mg of iron).

<u>(Example 19) Removing iron from (2R,2'R)-3,3'-dithiobis(2-amino-2-methylpropionic acid) (treatment with cellulose as filtering aid)</u>

10 g of the aqueous solution (10 g, containing 0.14 mg of iron) obtained in Example 17 was filtered through 100 mg of powdered cellulose (produced by Nippon Paper Chemicals, KC Flock) preliminarily washed with 0.01 M aqueous sodiumhydroxide solution, and further washed with 1 ml of 0.01 M aqueous sodium hydroxide solution to obtain 11 g of clear liquid. The iron content in the clear liquid was 3.8 ppm (corresponding to 0.04 mg of iron) .

<u>(Example 20) Preparation of (2R,2'R)-3,3'-dithiobis(2-amino-2-methylpropionic acid)</u>

In 313 g of water, 105 g of wet crystals of L-2.-amino-3-mercapto-2-methylpropionic acid hydrochloride obtained as in Reference Example 10 (the wet crystals containing 88 g of L-2-amino-3-mercapto-2-methylpropionic acid hydrochloride in terms of net content, 19 mg of iron, and 16 g of toluene) was dissolved, and the solution was concentrated under vacuum to 206 g. The toluene concentration in the solution was 28 ppm. Next, to the solution controlled to 25°C, 228 g of water and 170 g of 30 wt% aqueous sodium hydroxide solution were added over 30 minutes. The temperature of the solution increased to 35°C, and the pH of the solution reached 9.5. The reaction was then conducted for 12 hours

under vigorous stirring with air bubbling in the liquid while maintaining the inner temperature at 35°C to 40°C. The inner temperature was then cooled to 20°C to 25°C, and the reaction was further continued for 12 hours while maintaining this temperature. The resulting reaction solution (584 g) had a pH of 11.2 and reddish-brown crystals precipitated therein. L-2-Amino-3-mercapto-2-methylpropionic acid was not detected (less than 0.1% by HPLC-2).

The resulting reaction solution (281 g) was filtered through 2.07 g of activated charcoal (produced by Takeda Pharmaceutical Company Limited: activated charcoal Shirasagi A) preliminarily washed with 0.01 M aqueous sodium hydroxide solution, and further washed with 10 ml of 0.01 M aqueous sodium hydroxide solution to obtain 284 g of filtrate. Then, 280 g of the filtrate was neutralized with 31 g of concentrated hydrochloric acid at room temperature to adjust the pH to 4.5, and the precipitated crystals were filtered and washed with water to obtain 47.6 g of wet crystals containing 38.9 g of (2R,2'R)-3,3'-dithiobis(2-amino-2-methylpropionic acid). The iron content in the wet crystals was 2 ppm (corresponding to 0.1 mg of iron).

(Example 21) Preparation of L-2-amino-3-mercapto-2-methylpropionic acid by reduction of (2R,2'R) -3, 3'-dithiobis (2-amino-2-methylpropionic acid)

To 23.2 g of wet crystals of (2R,2'R)-3,3'-dithiobis(2-amino-2-methylpropionic acid) obtained in Example 20 (wet crystals containing 19.0 g of (2R,2'R)-3,3'-dithiobis(2-amino-2-methylpropionic acid) and 0.04 mg of iron) and 24 g of triphenylphosphine, 38 g of water, 19 g of concentrated hydrochloric acid, and 190 g of toluene were sequentially added at room temperature under nitrogen stream. The reaction was then conducted for 20 hours at 80°C. The residual ratio of (2R,2'R)-3,3'-dithiobis(2-amino-2-methylpropionic acid) in the resulting reaction solution was 0.1% (HPLC-3).

An aqueous solution obtained by separating the toluene layer from the reaction solution was washed with 190 g of toluene four times. As a result, neither triphenylphosphine nor triphenylphosphine oxide was detected in the aqueous solution (less than 0.01% by HPLC-3). An aqueous solution of L-2-amino-3-mercapto-2-methylpropionic acid hydrochloride having a purity of 99.9 area% (HPLC-2) was obtained thereby.

The aqueous solution was concentrated to 45 g (degree of vacuum: 30 to 60 mmHg, temperature:45°C), combined with 72 g of toluene, and subj ected to vacuum concentration (degree of vacuum: 40 to 60 mmHg, temperature: 40°C, distillation rate: 107 L/h·$m^2$) to bring the total weight to 60 g. The resulting solution was combined with 72 g of toluene and concentrated. The same operation was carried out a total of five times. Subsequently, toluene was added thereto to obtain 184 g of a toluene slurry of L-2-amino-3-mercapto-2-methylpropionic acid hydrochloride. Crystals were filtered, washed with toluene, and vacuum-dried to obtain 21.7 g of L-2-amino-3-mercapto-2-methylpropionic acid hydrochloride as a white solid. The purity of the crystals of the L-2-amino-3-mercapto-2-methylpropionic acid hydrochloride was 99.9 area% (HPLC-4), the iron content was 2 ppm (corresponding to 0.04 mg of iron), and the sodium content was 0.2 wt%. It was confirmed that the L-2-amino-3-mercapto-2-methylpropionic acid hydrochloride was more stable than L-2-amino-3-mercapto-2-methylpropionic acid hydrochloride obtained in Reference Example 10.

(Comparative Example 2) Preparation of 2-amino-3-mercapto-2-methylpropionic acid by reduction of 2-amino-3-benzylthio-2-methylpropionic acid

To 2,000mL of liquid ammonia cooled to -78°C, 11.3 g of 2-amino-3-benzylthio-2-methylpropionic acid was added, and 4.0 g of metallic sodium was slowly added over one hour. The resulting mixture was then warmed to -33°C and reacted for 1 hour, and ammonia was then distilled off by heating to room temperature. The resulting reaction solution was combined with 500 mL of deaerated water to conduct further concentration. Concentrated hydrochloric acid (25 g) was then added to obtain 82 g of an aqueous solution.

As a result of HPLC analysis (HPLC-2), the aqueous solution contained 8.0 g of L-2-amino-3-mercapto-2-methylpropionic acid hydrochloride and had a purity of 96.2 area%. Furthermore, 2-amino-3-benzylthio-2-methylpropionic acid was not detected (less than 0.1% by HPLC-1).

The aqueous solution (80 g) was concentrated to 16 g (degree of vacuum: 30 to 60 mmHg, temperature: 45°C), combined with 50 g of toluene, and subjected to vacuum concentration (degree of vacuum: 40 to 60 mmHg, temperature: 40°C, distillation rate: 107 L/h·$m^2$) to bring the total weight to 27 g. Toluene (50 g) was added thereto and the resulting mixture was concentrated. The same operation was carried out a total of five times, and then toluene was added to obtain 25 g of a toluene slurry of L-2-amino-3-mercapto-2-methylpropionic.acid hydrochloride. Crystals were filtered, washed with toluene, and vacuum-dried to obtain 11.8 g of L-2-amino-3-mercapto-2-methylpropionic acid hydrochloride as a white solid.

As a result of the HPLC analysis (HPLC-4), the content of L-2-amino-3-mercapto-2-methylpropionic acid hydrochloridein the crystals was 66 wt%, and the purity was 95.9 area%. The sodium content was 13 wt%.

Brief Description of Drawings

Fig. 1 is an IR spectrum of (5S,5'S)-5,5'-[dithiobis(methylene)]bis(5-methylhydantoin) obtained in Example 2.

Industrial Applicability

According to the present invention, in the production of an optically active R or S isomer of 2-amino-3-mercapto-2-methylpropionic acid derivative or salt thereof useful as an intermediate for pharmaceuticals and the like, a novel intermediate that can highly suppress contamination by various impurities can be obtained. By using this novel intermediate, it becomes possible to easily and efficiently produce a high purity optically active R or S isomer of a 2-amino-3-mercapto-2-methylpropionic acid derivative or salt thereof on an industrial production scale.

## Claims

1. A process of producing an optically active 2-amino-3-mercapto-2-methylpropionic acid derivative represented by general formula (1):

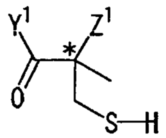

(1)

(wherein $Y^1$ is an unsubstituted hydroxyl group or a substituted or unsubstituted amino group and $Z^1$ is a substituted or unsubstituted amino group, or $Y^1$ and $Z^1$ together form a divalent group; and * is an asymmetric carbon) or salt thereof, the process comprising the steps of reducing an optically active 3,3'-dithiobis(2-amino-2-methylpropionic acid) derivative represented by general formula (2):

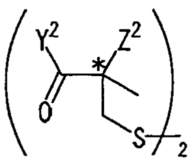

(2)

(wherein $Y^2$ and $Z^2$ respectively may be the same as or different from $Y^1$ and $Z^1$ above; $Y^2$ is an unsubstituted hydroxyl group or a substituted or unsubstituted amino group and $Z^2$ is a substituted or unsubstituted amino group, or $Y^2$ and $Z^2$ together form a divalent group; and * is an asymmetric carbon) or salt thereof so as to cleave the sulfur-sulfur bond; and converting $Y^2$ to $Y^1$ and/or $Z^2$ to $Z^1$ as necessary.

2. The production process according to claim 1, wherein one of metal hydride reagents, alkali metals, metals and acids, alkali metal sulfides, and phosphine compounds is used as a reductant in the reductive reaction.

3. The production process according to claim 1 or 2, wherein the reductive reaction is conducted in the presence of an acidic substance.

4. A process for producing an optically active 2-amino-3-mercapto-2-methylpropionic acid derivative represented by said general formula (1) or salt thereof, the process comprising a step of removing impurities in an aqueous medium solution containing the optically active 2-amino-3-mercapto-2-methylpropionic acid derivative(1)orsalt thereof produced by the process according to any one of claims 1 to 3 into an organic solvent phase immiscible with the aqueous medium so as to purify the optically active 2-amino-3-mercapto-2-methylpropionic acid derivative (1) or salt thereof.

5. The production process according to claim 4, wherein the organic solvent immiscible with the aqueous medium is

an aromatic hydrocarbon solvent.

**6.** The production process according to claim 4 or 5, wherein the pH of the aqueous medium solution is adjusted out of the range of 4 to 5.

**7.** The production process according to any one of claims 4 to 6, wherein the pH of the aqueous medium solution is adjusted to 3 or less.

**8.** The production process according to any one of claims 1 to 7, wherein $Y^1$ is an unsubstituted hydroxyl group and $Z^1$ is an unsubstituted amino group.

**9.** The production process according to any one of claims 1 to 8, wherein $Y^2$ is $Y^1$ and $Z^2$ is $Z^1$.

**10.** The production process according to any one of claims 1 to 9, wherein a compound prepared by forming a sulfur-sulfur bond between two molecules of an optically active 3-mercapto-2-methylpropionic acid derivative represented by general formula (3):

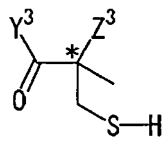

(3)

(wherein $Y^3$ and $Z^3$ may respectively be the same as or different from $Y^2$ and $Z^2$ above; $Y^3$ is an unsubstituted hydroxyl group or a substituted or unsubstituted amino group and $Z^3$ is a substituted or unsubstituted amino group, or $Y^3$ and $Z^3$ together form a divalent group; and * is an asymmetric carbon) or salt thereof and converting $Y^3$ to $Y^2$ and/or $Z^3$ to $Z^2$ as necessary is used as the optically active 3,3'-dithiobis (2-amino-2-methylpropionic acid) derivative represented by said formula (2) or salt thereof.

**11.** The production process according to claim 10, wherein the sulfur-sulfur bond is formed by oxidation using an oxidant.

**12.** The production process according to claim 11, wherein the oxidant is oxygen.

**13.** The production process according to claim 12, wherein an ionic iron compound is used as an oxidation catalyst.

**14.** The production process according to any one of claims 10 to 13, wherein $Y^3$ is an unsubstituted hydroxyl group and $Z^3$ is an unsubstituted amino group.

**15.** The production process according to any one of claims 10 to 13, wherein $Y^3$ and $Z^3$ together form ureylene group (-NHCONH-).

**16.** The production process according to any one of claims 10 to 15, wherein $Y^2$ is an unsubstituted hydroxyl group and $Z^2$ is an unsubstituted amino group.

**17.** The production process according to any one of claims 10 to 15, wherein $Y^3$ is $Y^2$ and $Z^3$ is $Z^2$.

**18.** The production process according to any one of claims 10 to 16, wherein $Y^3$ is converted to $Y^2$ and/or $Z^3$ is converted to $Z^2$ by hydrolysis after the formation of the sulfur-sulfur bond.

**19.** The production process according to claim 18, wherein the hydrolysis is conducted under acidic conditions.

**20.** The production process according to any one of claims 1 to 9, wherein the optically active 3,3'-dithiobis(2-amino-2-methylpropionic acid) derivative represented by said general formula (2) or salt thereof is contained in the optically active 2-amino-3-mercapto-2-methylpropionic acid derivative represented by said general formula (1) or salt thereof.

**21.** The production process according to claim 20, wherein the content of the optically active 3,3'-dithiobis(2-amino-2-methylpropionic acid) derivative represented by said general formula (2) or salt thereof in the optically active 2-amino-3-mercapto-2-methylpropionic acid derivative represented by said general formula (1) or salt thereof is 0.1% or more.

**22.** The production process according to any one of claims 10 to 19, wherein the optically active 3-mercapto-2-methylpropionic acid derivative represented by said general formula (3) or salt thereof is contained in the optically active 3,3'-dithiobis(2-amino-2-methylpropionic acid) derivative represented by said general formula (2) or salt thereof.

**23.** The production process according to claim 22, wherein the content of the optically active 3-mercapto-2-methylpropionic acid derivative represented by said general formula (3) or salt thereof in the optically active 3,3'-dithiobis(2-amino-2-methylpropionic acid) derivative represented by said general formula (2) or salt thereof is 0.1% or more.

**24.** A process for producing an optically active 2-amino-3-mercapto-2-methylpropionic acid derivative representedby said general formula (1) or salt thereof, comprising performing crystallization in the presence of an organic solvent from an aqueous medium solution containing an optically active 2-amino-3-mercapto-2-methylpropionic acid derivative represented by said general formula (1) or salt thereof produced by the method according to any one of claims 1 to 23.

**25.** The production process according to claim 24, wherein a salt with an acid of the optically active 2-amino-3-mercapto-2-methylpropionic acid derivative (1) is crystallized.

**26.** The production process according to claim 24 or 25, wherein concentration is conducted in the presence of the organic solvent to replace water by the organic solvent while removing water from the system, and thereby crystallize the compound.

**27.** The production process according to any one of claims 24 to 26, wherein the organic solvent immiscible with the aqueous medium is used.

**28.** The production process according to any one of claims 24 to 27, wherein the concentration and the solvent replacement are conducted until the amount of the residual water is 100 wt% or less relative to the optically active 2-amino-3-mercapto-2-methylpropionic acid derivative (1) or salt thereof to thereby conduct crystallization.

**29.** The production process according to any one of claims 26 to 28, wherein an evaporation rate during the concentration is controlled to 1,000 $L/h \cdot m^2$ or less.

**30.** A process of producing an optically active 3,3'-dithiobis(2-amino-2-methylpropionic acid) derivative represented by general formula (2):

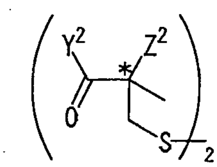

(2)

(wherein $Y^2$ is an unsubstituted hydroxyl group or a substituted or unsubstituted amino group and $Z^2$ is a substituted or unsubstituted amino group, or $Y^2$ and $Z^2$ together form a divalent group; and * is an asymmetric carbon) or salt thereof, the process comprising the steps of oxidizing an optically active 3-mercapto-2-methylpropionic acid derivative represented by general formula (3):

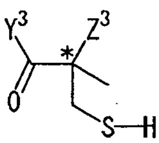

(3)

(wherein $Y^3$ and $Z^3$ may respectively be the same as or different from $Y^2$ and $Z^2$ above; $Y^3$ is an unsubstituted hydroxyl group or a substituted or unsubstituted amino group and $Z^3$ is a substituted or unsubstituted amino group, or $Y^3$ and $Z^3$ together form a divalent group; and * is an asymmetric carbon) or salt thereof to form a sulfur-sulfur bond between two molecules and converting $Y^3$ to $Y^2$ and/or $Z^3$ to $Z^2$ as necessary.

**31.** The production process according to claim 30, wherein the oxidation is oxygen oxidation.

**32.** The production process according to claim 31, wherein an ionic iron compound is used as an oxidation catalyst.

**33.** The production process according to any one of claims 30 to 32, wherein $Y^3$ is an unsubstituted hydroxyl group and $Z^3$ is an unsubstituted amino group.

**34.** The production process according to any one of claims 30 to 32, wherein $Y^3$ and $Z^3$ together form ureylene group (-NHCONH-).

**35.** The production process according to any one of claims 30 to 34, wherein $Y^2$ is an unsubstituted hydroxyl group and $Z^2$ is an unsubstituted amino group.

**36.** The production process according to any one of claims 30 to 34, wherein $Y^3$ is $Y^2$ and $Z^3$ is $Z^2$.

**37.** The production process according to any one of claims 30 to 35, wherein $Y^3$ is converted to $Y^2$ and/or $Z^3$ is converted to $Z^2$ by hydrolysis after the formation of the sulfur-sulfur bond.

**38.** The production process according to claim 37, wherein the hydrolysis is conducted under acidic conditions.

**39.** The production process according to any one of claims 30 to 38, wherein the optically active 3-mercapto-2-methylpropionic acid derivative represented by said general formula (3) or salt thereof is contained in the optically active 3,3'-dithiobis(2-amino-2-methylpropionic acid) derivative represented by said general formula (2) or salt thereof.

**40.** A process for purifying an optically active 3,3'-dithiobis(2-amino-2-methylpropionic acid) derivative represented by general formula (2):

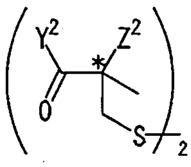
(2)

(wherein $Y^2$ is an unsubstituted hydroxyl group or a substituted or unsubstituted amino group and $Z^2$ is a substituted or unsubstituted amino group, or $Y^2$ and $Z^2$ together form a divalent group; and * is an asymmetric carbon) or salt thereof, the process comprising adjusting an aqueous medium solution containing the optically active 3,3'-dithiobis (2-amino-2-methylpropionic acid) derivative (2) or salt thereof to be basic so as to separate and remove impurities from the solution.

**41.** The purification process according to claim 40, wherein the pH of the aqueous medium solution adjusted to be basic is 10 or more.

**42.** The purification process according to claim 40 or 41, wherein the precipitated impurities are removed by filtration.]

**43.** A process for purifying an optically active 3,3'-dithiobis(2-amino-2-methylpropionic acid) derivative represented by general formula (2):

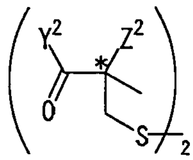

(wherein $Y^2$ is an unsubstituted hydroxyl group or a substituted or unsubstituted amino group and $Z^2$ is a substituted or unsubstituted amino group, or $Y^2$ and $Z^2$ together form a divalent group; and * is an asymmetric carbon), the process comprising adjusting an aqueous medium solution containing the optically active 3,3'-dithiobis(2-amino-2-methylpropionic acid) derivative (2) or salt thereof, to be neutral to acidic so as to crystallize the optically active 3,3'-dithiobis(2-amino-2-methylpropionic acid) derivative (2) and remove impurities.

**44.** The purification process according to claim 43, wherein the pH of the aqueous medium solution adjusted to be neutral to acidic is 9 or less.

**45.** A process for purifying a salt with an acid of an optically active 3,3'-dithiobis(2-amino-2-methylpropionic acid) derivative represented by general formula (2):

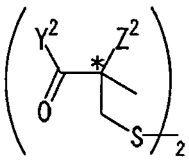

(wherein $Y^2$ is an unsubstituted hydroxyl group or a substituted or unsubstituted amino group and $Z^2$ is a substituted or unsubstituted amino group, or $Y^2$ and $Z^2$ together form a divalent group; and * is an asymmetric carbon), the process comprising adjusting an aqueous medium solution containing the optically active 3,3'-dithiobis(2-amino-2-methylpropionic acid) derivative or salt thereof to be highly acidic so as to crystallize the salt with the acid of the optically active 3,3'-dithiobis(2-amino-2-methylpropionic acid) derivative (2) and remove impurities.

**46.** The purification process according to claim 45, wherein the pH of the aqueous medium solution adjusted to be highly acidic is 1 or less.

**47.** The purification process according to any one of claims 40 to 46, wherein the impurities comprise an inorganic substance.

**48.** The purification process according to claim 47, wherein the inorganic substance is an ionic compound of a heavy metal.

**49.** The production process according to any one of claims 1 to 9, wherein the optically active 3,3'-dithiobis(2-amino-2-methylpropionic acid) derivative represented by said formula (2) or salt thereof purified by the process according to any one of claims 40 to 48 is used.

**50.** A process of producing a compound represented by said general formula (2), comprising purifying the compound represented by said formula (2) produced by the process according to any one of claims 30 to 39 by the purification process according to any one of claims 40 to 48.

**51.** An optically active (2R,2'R) or (25,2'S)-3,3'-dithiobis(2-amino-2-methylpropionic acid) derivative represented by general formula (4):

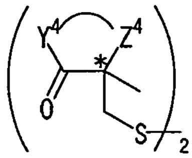 (4)

(wherein $-Y^4-Z^4-$ is a divalent group; and * is an asymmetric carbon) or salt thereof.

**52.** The optically active (2R,2'R) or (2S,2'S)-3,3'-dithiobis(2-amino-2-methylpropionic acid) derivative or salt thereof according to claim 51, wherein $-Y^4-Z^4-$ is a substituted or unsubstituted ureylene group (-NHCONH-).

Fig. 1

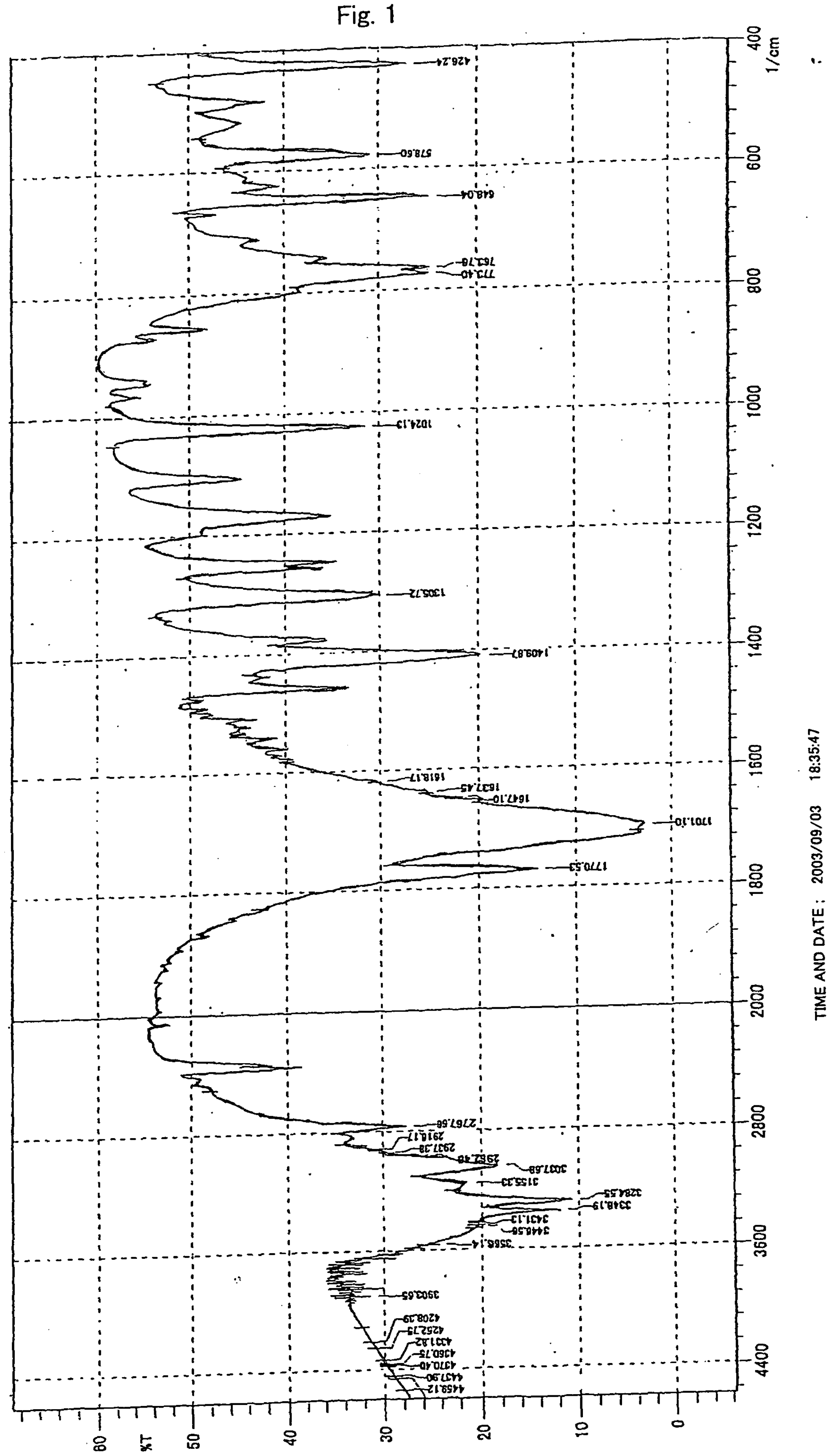

%T
1/cm
TIME AND DATE ; 2003/09/03 18:35:47
INTEGRATED QUANTITY ; 40
DECOMPOSITION ; 4 [1/cm]
APODIZATION ; Happ-Genzel

**INTERNATIONAL SEARCH REPORT**

International application No.
PCT/JP2004/012157

A. CLASSIFICATION OF SUBJECT MATTER
Int.Cl[7] C07C319/06, 323/58, C07B53/00, C07D233/76, C12P11/00

According to International Patent Classification (IPC) or to both national classification and IPC

B. FIELDS SEARCHED

Minimum documentation searched (classification system followed by classification symbols)
Int.Cl[7] C07C319/06, 323/58, C07B53/00, C07D233/76, C12P11/00

Documentation searched other than minimum documentation to the extent that such documents are included in the fields searched

Electronic data base consulted during the international search (name of data base and, where practicable, search terms used)
REGISTRY(STN), CA(STN)

C. DOCUMENTS CONSIDERED TO BE RELEVANT

| Category* | Citation of document, with indication, where appropriate, of the relevant passages | Relevant to claim No. |
|---|---|---|
| P,X | JP 2004-43309 A (Showa Denko Kabushiki Kaisha), 12 February, 2004 (12.02.04), Claims; pages 27 to 35 (Family: none) | 1-52 |
| X | KEDROWSKI, Bant L. et al., Thiazoline ring formation from 2-methylcysteines and 2-halomethylalanines, Heterocycles, 2002, 58, 601-634 | 1-52 |
| X | DELLA Monica, Carmela et al., Structural revision of halipeptins: synthesis of the theiazoline unit and isolation halipeptin C, Tetrahedron Letters, 2002, 43(33), 5707-5710 | 1-52 |

☐ Further documents are listed in the continuation of Box C. ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 09 November, 2004 (09.11.04) | 30 November, 2004 (22.11.04) |
| Name and mailing address of the ISA/ Japanese Patent Office | Authorized officer |
| Facsimile No. | Telephone No. |

Form PCT/ISA/210 (second sheet) (January 2004)